# EUROPEAN PATENT APPLICATION

(11) **EP 3 473 702 A1**
(43) Date of publication of application: **24.04.2019**
(21) Application number: 17815134.6
(22) Date of filing: 01.06.2017
(51) Int. Cl.: C12M 3/00, C12M 1/00, B65D 77/06, G01N 1/28

(54) **CONTAINER FOR TRANSPORTING CELLS AND BIOTISSUE**

(30) Priority: 21.06.2016 JP 2016122315
(71) Applicant: Sanplatec Corporation Ltd., Osaka 530-0035 (JP); Riken, Wako-shi, Saitama 351-0198 (JP)
(72) Inventor: KATOU Satoshi, Osaka-shi Osaka 530-0035 (JP); KUWABARA Junichi, Osaka-shi Osaka 530-0035 (JP); TAKAHASHI Masayo, Wako-shi Saitama 351-0198 (JP); KOIDE Naoshi, Wako-shi Saitama 351-0198 (JP)
(74) Representative: Boult Wade Tennant LLP
(86) International application number: PCT/JP2017/020473
(87) International publication number: WO 2017/221665

(57) **Abstract**

The invention provides a cell and biotissue transporting container for transporting cells or biotissues while maintaining their cultured state. A cell/biotissue transporting container A10 includes a base member 1 with an opening 110 at an upper end and a first tubular portion 11 extending vertically, a lid body 2 including a top plate portion 21 to close the opening 110 and a second tubular portion 22 extending from a peripheral edge of the top plate portion 21 in the thickness direction to fit onto the first tubular portion 11, and a container body 3, formed separately from the base member 1 using a flexible material, where the container body includes a bottom wall portion 31, a tubular side wall portion 32 rising from a peripheral edge of the bottom wall portion 31 and inserted into the opening 110, and a flange 33 extending outward from an upper edge of the side wall portion 32. At least one of the first and second tubular portions 11 and 22 includes a lock preventing relative movement between the base member 1 and the lid body 2 with the flange 33 being clamped between a peripheral edge portion of the opening 110 of the base member and the top plate portion 21 of the lid body 2.

## Description

### TECHNICAL FIELD

The present invention relates to a technology for transporting cells or biological tissues, and more particularly relates to a container suitable for transporting cells or biological tissues while maintaining the cultured state of the cells or the biological tissues.

### BACKGROUND ART

Despite recent advances in development of cell preparations for use in regenerative medicine, methods for transporting them have not been established yet. In order to transport living cells from a facility in which they are produced to a medical facility safely without freezing them, it is necessary to transport them while avoiding vibrations and shocks as well as preventing leakage and contamination, and for some types of cells, also while maintaining ventilated conditions at a suitable temperature. Accordingly, establishment of such a transportation system and realization of practical utilization thereof are matters that require immediate attention. Conventional general purpose laboratory containers are not applicable as transportation containers in which living cells are placed directly.

As general purpose containers that have been used for culturing cells and biological tissues, petri dishes (receivers), well plates, probes, and the like are known, for example. These culture containers are designed for culture under ventilated conditions. Accordingly, even if such a culture container has a lid, the lid is designed so as to be merely placed on the culture container and cannot seal the culture container. A method commonly employed to seal the culture container is, for example, to attach a film with adhesive to an upper edge portion of the tubular wall of the container. Also, covering the upper edge portion of the tubular wall of the container using an elastomer sheet as a lid has been proposed (see Patent Document 1, for example).

In the case where an opening of the culture container is closed using the above-described film with adhesive for transportation of the culture container, although the culture container may be sealed, the culture medium may come into direct contact with the adhesive surface of the film. Accordingly, there is a risk of contamination resulting from elution of the adhesive. In addition, the contents of the culture container may spill out of the container in reaction to the action of removing the adhesive film. The method of covering the container using the elastomer sheet as a lid does not achieve sufficiently reliable liquid sealing performance during the transportation.

For a well plate as one type of culture container, there has been developed a technology for preparing a cell sheet for use in regenerative medicine using a device called a cell culture insert to be set in the well plate. However, also for a device on which a cell sheet is overlaid, a container suitable for transporting such a device stably has not been provided yet. If the cell sheet peels away from the device, it is difficult for the cell sheet to retain its sheet-like shape. Accordingly, there is a demand for a transportation container that holds the device stably, prevents detachment of the cell sheet by filling the container with a culture medium so as to restrict shaking of the culture medium, and exhibits sufficient liquid sealing performance.

### PRIOR ART DOCUMENTS

### PATENT DOCUMENT

Patent Document 1: JP 2002-159284A

### DISCLOSURE OF THE INVENTION

### PROBLEM TO BE SOLVED BY THE INVENTION

The present invention was devised in light of the above-described circumstances, and a main object of the present invention is to provide a cell and biological tissue transporting container suitable for transporting cells or a biological tissue while maintaining the cultured state of the cells or the biological tissue.

### MEANS FOR SOLVING THE PROBLEM

In order to solve the above-described problems, the present invention employs the following technical solutions.

A first aspect of the present invention provides a cell and biological tissue transporting container including: a base member including an opening at an upper end and a first tubular portion that extends vertically; a lid body including a top plate portion to close the opening and a second tubular portion that extends from an outer peripheral side of this top plate portion in a thickness direction of the top plate portion and fits onto the first tubular portion of the base member; and a container body formed separately from the base member using a flexible material, the container body including a bottom wall portion, a tubular side wall portion that rises from a peripheral edge of this bottom wall portion and is inserted into the opening, and a flange that extends outward from an upper edge of this side wall portion. The base member and the lid body are provided with a lock that prevents relative movement between the base member and the lid body with the flange of the container body being clamped between a peripheral edge portion of the opening of the base member and the top plate portion of the lid body.

In a preferred embodiment, the side wall portion of the container body includes a large-diameter portion that is located closer to the flange and a small-diameter portion that is located closer to the bottom wall portion and has a smaller radial dimension than the large-diameter portion.

In a preferred embodiment, an outer surface of the small-diameter portion is provided with a plurality of outer surface projections that are disposed radially as viewed in a vertical direction and each of which can abut against an inner surface of the first tubular portion of the base member.

In a preferred embodiment, an outer surface of the side wall portion of the container body abuts against an inner surface of the first tubular portion of the base member while opposing the inner surface.

In a preferred embodiment, the lock includes an external thread that is provided in one of the first tubular portion and the second tubular portion and an internal thread that is provided in the other one of the first tubular portion and the second tubular portion and is threadedly engageable with the external thread.

In a preferred embodiment, the flange of the container body is constituted by a thick portion that is relatively thick, and at least one of the bottom wall portion and the side wall portion of the container body includes a thin portion that is relatively thin and gas permeable.

In a preferred embodiment, the base member includes a bottom portion that is connected to a lower end of the first tubular portion or a vicinity thereof and extends inwardly of the first tubular portion as viewed in a vertical direction.

In a preferred embodiment, the bottom portion of the base member extends inwardly of the first tubular portion from a position displaced upward from the lower end of the first tubular portion and has a through hole that passes through the base member in a thickness direction, and a slit is formed at the lower end of the first tubular portion.

In a preferred embodiment, the bottom portion of the base member closes an internal space of the first tubular portion, and the top plate portion of the lid body has a through hole that passes through the top plate portion in a thickness direction.

In a preferred embodiment, the flange of the container body is constituted by a thick portion that is relatively thick, and the bottom wall portion of the container body includes a thin portion that is relatively thin and transparent.

In a preferred embodiment, the base member includes a bottom portion that is connected to a lower end of the first tubular portion or a vicinity thereof and closes an internal space of the first tubular portion.

In a preferred embodiment, the lid body includes a protruding portion that is accommodated inside the side wall portion of the container body and protrudes toward the bottom wall portion of the container body.

In a preferred embodiment, the lid body includes a small-diameter tubular nozzle portion that protrudes upward and a cap that can close a tip of the nozzle portion.

In a preferred embodiment, the lid body includes: an inner lid including the top plate portion; and an outer lid including a presser portion that is stacked on the top plate portion as viewed in a thickness direction of the top plate portion and the second tubular portion that is connected to an outer peripheral edge of the presser portion.

In a preferred embodiment, the outer lid has an opening hole formed inside the presser portion, and the inner lid is provided with ports that are configured such that the ports overlap the opening hole as viewed in the thickness direction of the top plate portion, upper ends thereof are open to an outside, and lower ends thereof are open to an internal space of the container body.

In a preferred embodiment, in the inner lid, a first port, a second port, a third port, and a fourth port are provided as the ports, the first and second ports are provided in the vicinity of the side wall portion of the container body, and the third and fourth ports are provided farther inward in a radial direction of the top plate portion than the first and second ports.

In a preferred embodiment, at least a central portion of the inner lid is transparent, and the third and fourth ports are provided at positions displaced from the central portion so as to avoid the central portion.

In a preferred embodiment, the transparent central portion of the inner lid has a recessed shape recessed downward.

In a preferred embodiment, the bottom wall portion of the container body is provided with one or more protrusions that protrude upward.

In a preferred embodiment, the one or more protrusions are in a substantially circular shape or in arc shapes that constitute parts of the same circle as viewed in a vertical direction.

In a preferred embodiment, the side wall portion of the container body includes an overhanging portion that overhangs farther inward than a remaining portion as viewed in a vertical direction.

In a preferred embodiment, the side wall portion of the container body is provided with a plurality of ribs each of which extends inward and that are disposed radially as viewed in a vertical direction.

Other characteristics and advantages of the present invention will become more apparent by the following detailed description with reference to the accompanying drawings.

### BRIEF DESCRIPTION OF DRAWINGS

FIG. 1 is a perspective view showing a first embodiment of the cell and biological tissue transporting container according to the present invention.
FIG. 2 is an exploded perspective view of the cell and biological tissue transporting container shown in FIG. 1.
FIG. 3 is a perspective view of the cell and biological tissue transporting container shown in FIG. 1 in a state where a lid body is removed therefrom.
FIG. 4 is an enlarged vertical sectional view of the cell and biological tissue transporting container shown in FIG. 1.
FIG. 5 is a perspective view of a container body.
FIG. 6 is a plan view of the container body.
FIG. 7 is a sectional view taken in the same manner as FIG. 4 and showing a state where contents are in the container body.
FIG. 8 is an exploded perspective view showing a modified example of the cell and biological tissue transporting container shown in FIG. 1.
FIG. 9 is a perspective view showing the cell and biological tissue transporting container shown in FIG. 8 in a state where a lid body is removed therefrom.
FIG. 10 is an enlarged vertical sectional view of the cell and biological tissue transporting container shown in FIG. 8 in an assembled state.
FIG. 11 is a cross-sectional end view taken along line XI-XI in FIG. 10.
FIG. 12 is a perspective view of a container body.
FIG. 13 is a plan view of the container body.
FIG. 14 is a sectional view taken in the same manner as FIG. 10 and showing a state where contents are in the container body.
FIG. 15 is an enlarged vertical sectional view showing another modified example of the cell and biological tissue transporting container shown in FIG. 1.
FIG. 16 is an enlarged vertical sectional view showing still another modified example of the cell and biological tissue transporting container shown in FIG. 1.
FIG. 17 is an enlarged vertical sectional view showing still another modified example of the cell and biological tissue transporting container shown in FIG. 1.
FIG. 18 is a plan view of a container body in the cell and biological tissue transporting container shown in FIG. 17.
FIG. 19 is an enlarged vertical sectional view showing still another modified example of the cell and biological tissue transporting container shown in FIG. 1.
FIG. 20 is an enlarged vertical sectional view showing still another modified example of the cell and biological tissue transporting container shown in FIG. 1.
FIG. 21 is an enlarged vertical sectional view showing still another modified example of the cell and biological tissue transporting container shown in FIG. 1.
FIG. 22 is a bottom view of a container body in the cell and biological tissue transporting container shown in FIG. 21.
FIG. 23 is an enlarged vertical sectional view showing still another modified example of the cell and biological tissue transporting container shown in FIG. 1.
FIG. 24 is a perspective view showing a second embodiment of the cell and biological tissue transporting container according to the present invention.
FIG. 25 is an exploded perspective view of the cell and biological tissue transporting container shown in FIG. 24.
FIG. 26 is a plan view of the cell and biological tissue transporting container shown in FIG. 24.
FIG. 27 is a sectional view taken along line XXVII-XXVII in FIG. 26.
FIG. 28 is a cross-sectional end view taken along line XXVIII-XXVIII in FIG. 27.
FIG. 29 is a sectional view taken in the same manner as FIG. 27 and showing an example of a state where contents are in the container body.
FIG. 30 is a sectional view taken in the same manner as FIG. 27 and showing another example of a state where contents are in the container body.
FIG. 31 is a perspective view showing another example of a member on which cultured cells are to be placed.
FIG. 32 is a vertical sectional view of the member shown in FIG. 31.
FIG. 33 is a perspective view showing a third embodiment of the cell and biological tissue transporting container according to the present invention.
FIG. 34 is an exploded perspective view of the cell and biological tissue transporting container shown in FIG. 33.
FIG. 35 is an enlarged vertical sectional view of the cell and biological tissue transporting container shown in FIG. 33.
FIG. 36 is an exploded perspective view showing a modified example of the cell and biological tissue transporting container shown in FIG. 33.
FIG. 37 is an enlarged vertical sectional view of the cell and biological tissue transporting container shown in FIG. 36 in an assembled state.
FIG. 38 is a plan view showing a cell and biological tissue transporting container according to a reference example of the present invention.
FIG. 39 is an exploded perspective view of the cell and biological tissue transporting container shown in FIG. 38.
FIG. 40 is a sectional view taken along line XL-XL in FIG. 38.

### MODES FOR CARRYING OUT THE INVENTION

Preferred embodiments of the present invention will be described specifically below with reference to the drawings.

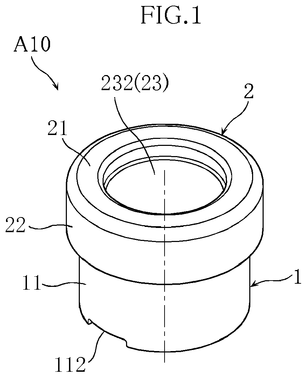

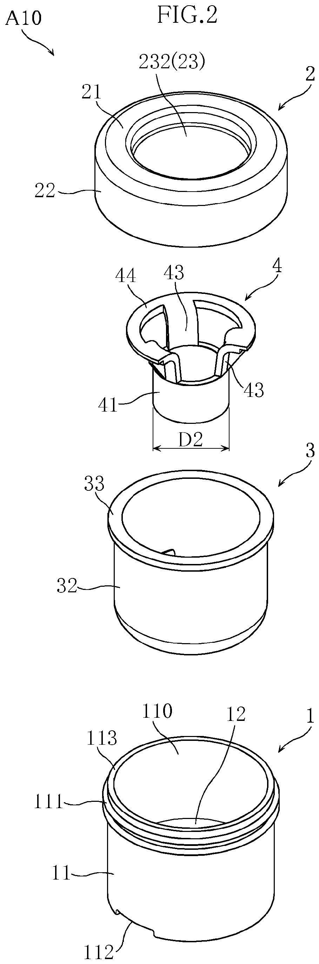

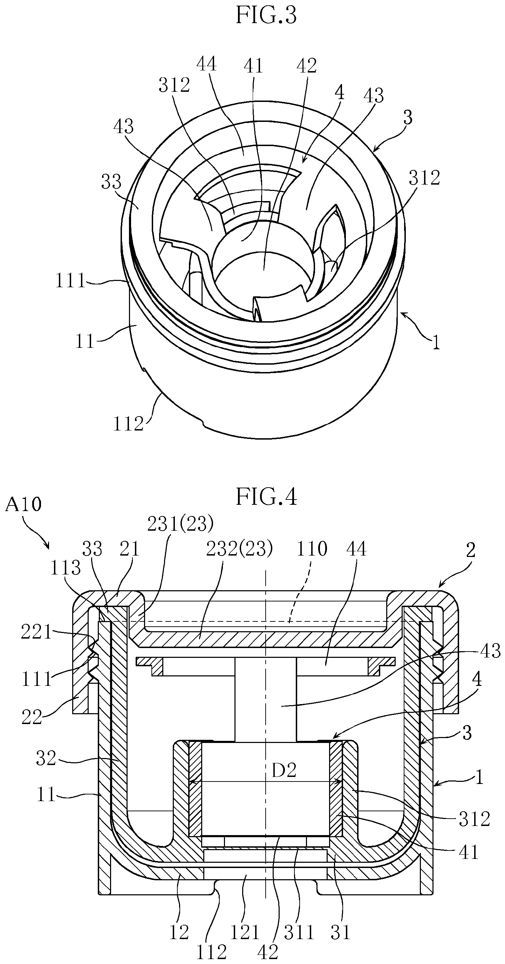

FIGS. 1 to 4 show the first embodiment of the cell and biological tissue transporting container according to the present invention. A cell and biological tissue transporting container A10 of the present embodiment includes a base member 1, a lid body 2, and a container body 3. FIGS. 1 and 4 show an assembled state in which the base member 1, the lid body 2, and the container body 3 are combined with one another, and this will be described more specifically below. In the present embodiment, as can be seen in FIGS. 3 and 4, a device 4 is held by the container body 3. FIG. 2 is a perspective view showing the components of the cell and biological tissue transporting container A10 in a disassembled state. FIG. 3 is a perspective view showing a state where the lid body 2 is removed.

In the present embodiment, the base member 1 is in the form of a container including a first tubular portion 11 and a bottom portion 12 and having an opening 110 at an upper end of the first tubular portion 11. The first tubular portion 11 is generally cylindrical, and an external thread 111 is formed on an upper outer peripheral surface of the first tubular portion 11. A slit 112 is formed at the lower end of the first tubular portion 11. In the present embodiment, the bottom portion 12 has a circular through hole 121 that passes through the bottom portion 12 in the thickness direction at a central portion thereof. The significance of the through hole 121 will be described below.

The lid body 2 has a top plate portion 21, a second tubular portion 22, and a protruding portion 23, and is a component for sealing the container body 3. The outer shape of the top plate portion 21 is generally circular, and a flange 33 (to be described below) of the container body 3 is clamped between this top plate portion 21 and an upper end portion 113 (a peripheral edge portion of the upper end opening) of the first tubular portion 11 of the base member 1. The second tubular portion 22 extends from the peripheral edge of the top plate portion 21 in the thickness direction of the top plate portion 21 (downward in the drawings), and is generally cylindrical. An internal thread 221 is formed on the inner peripheral surface of the second tubular portion 22, and the internal thread 221 is threadedly engageable with the external thread 111 of the base member 1 (the first tubular portion 11) . As can be seen in FIG. 4, the protruding portion 23 is accommodated inside a side wall portion 32 (to be described below) of the container body 3, and protrudes in the vertical direction toward a bottom wall portion 31 of the container body 3. In the present embodiment, the protruding portion 23 has an inner cylindrical portion 231 that extends downward from the top plate portion 21 and a bottom plate portion 232 that closes the lower end of the inner cylindrical portion 231.

The base member 1 and the lid body 2 are formed of a translucent or transparent plastic material, for example. As such a plastic material, a plastic material having transparency is suitable, and examples thereof include, but not limited to, polystyrene and methylpentene, and in addition, polycarbonate, cycloolefin polymers, and cycloolefin copolymers.

The container body 3 is a component for containing cultured cells and a culture medium therein. The container body 3 is formed of a flexible and resilient material. Examples of the material of the container body 3 include silicone rubber, natural rubber, urethane rubber, and elastomer resin. In light of the contact between the container body 3 and the contents (the cultured cells and the culture medium) to be described specifically below, it is more preferable that the material of the container body 3 is medical grade silicone rubber, which is free of cytotoxicity and has biocompatibility. Regarding the hardness of the container body 3, it is preferable that the container body 3 has a rubber hardness of about 20 to 40 degrees.

The container body 3 is a rubber molded product, for example, and as shown in FIGS. 4 to 6, the container body 3 has the bottom wall portion 31, the side wall portion 32 that rises from the peripheral edge of the bottom wall portion 31, and the flange 33. The bottom wall portion 31 is substantially circular in a plan view. The side wall portion 32 is generally cylindrical. The flange 33 is generally annular, and extends radially outward from the upper edge of the side wall portion 32. The flange 33 has an appropriate thickness and appropriate elastic resilience against the loads applied from above and below. The flange 33 has a thickness of about 1 to 3 mm, for example. The flange 33 corresponds to the thick portion of the present invention. In the assembled state shown in FIG. 4, the outer surface of the side wall portion 32 abuts against the inner surface of the first tubular portion 11 of the base member 1 while opposing the inner surface. In the assembled state, the side wall portion 32 is inserted into the opening 110 of the base member 1, and the side wall portion 32 and the bottom wall portion 31 are placed inside the first tubular portion 11 of the base member 1 by the insertion.

The bottom wall portion 31 is provided with protrusions 312. The protrusions 312 extend upward, and the multiple (two in the present embodiment) protrusions are provided. These protrusions 312 are provided in such a manner that they are in arc shapes that constitute parts of the same circle in a plan view (i.e., as viewed in the vertical direction) .

As shown in FIG. 4, in the present embodiment, the bottom wall portion 31 has a thin portion 311 provided in a central portion thereof. As clear from FIG. 4, the thin portion 311 has a smaller thickness than other portions. The thin portion 311 has a thickness of about 0.2 to 0.3 mm, for example. The thin portion 311 is gas permeable. When viewed in the vertical direction, the thin portion 311 is substantially circular and is surrounded by the circle (same circle) constituted by the multiple protrusions 312.

The device 4 to be held by the container body 3 is, for example, a device generally called a cell culture insert, which is used for carrying out a predetermined cell culture method. The device 4 has a culture portion 41, a thin film 42, coupling portions 43, and a ring-shaped portion 44. The culture portion 41 is a cylindrical portion for containing cultured cells, and the lower end of this culture portion 41 is closed by the thin film 42. The thin film 42 is used as a culture surface on which cultured cells are to be placed, and the cultured cells on the thin film 42 are immersed in a culture medium. The thin film 42 is in a film shape having a thickness of about 0.2 to 10 µm, for example, and the culture medium to be used for cell culture can pass through the thin film 42. The coupling portions 43 couple the culture portion 41 and the ring-shaped portion 44 in a state where the culture portion 41 and the ring-shaped portion 44 are apart from each other. In the present embodiment, the ring-shaped portion 44 is coupled to the culture portion 41 via the three coupling portions 43 extending from the upper end of the culture portion 41. The ring-shaped portion 44 has a partially cutaway annular shape in a plan view.

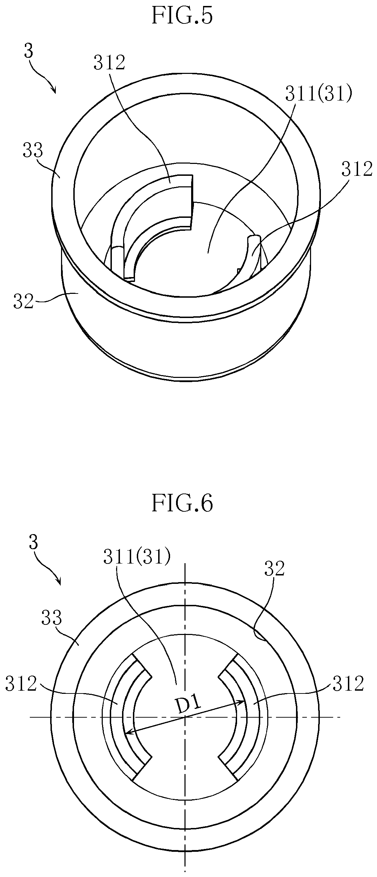

The device 4 with the above-described configuration is typically set in a culture container called a well plate to perform cell culture. In the present embodiment, the inner diameter dimension D1 (see FIG. 6) of the circle constituted by the multiple protrusions 312 in the above-described container body 3 is substantially the same as the outer diameter dimension D2 (see FIGS. 2 and 4) of the culture portion 41 (the cylindrical portion) or slightly smaller than the outer diameter dimension D2. Accordingly, by inserting the culture portion 41 inside the multiple protrusions 312, the device 4 is held in a predetermined orientation inside the container body 3, as shown in FIGS. 3 and 4 .

Next, usage and functions of the cell and biological tissue transporting container A10 will be described.

The cell and biological tissue transporting container A10 is used for containing cultured cells or a biological tissue in the container body 3 together with a culture medium and transporting the cultured cells or the biological tissue while maintaining the cultured state (cultured state-maintaining transportation). The cells, biological tissue, and culture medium to be contained in the container body 3 are not particularly limited.

For example, a cell preparation for use in regenerative medicine can be contained in the form of a suspension or a cell sheet overlaid on the device. When transporting the cell suspension or cell sheet in a non-frozen state, in order to prevent the culture medium from being shaken to damage the cells, it is necessary to fill the container body 3 completely with the culture medium to restrict the movement of the culture medium.

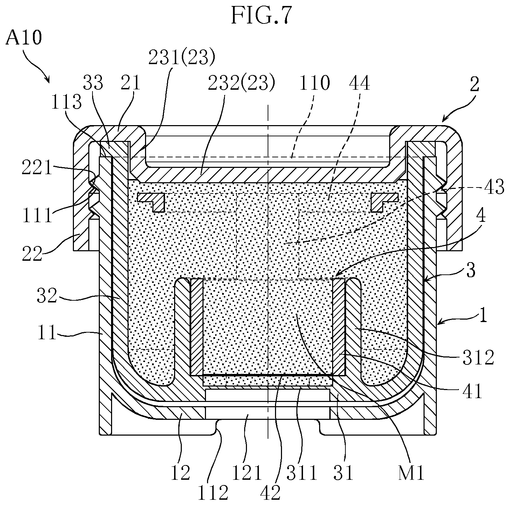

FIG. 7 shows the assembled state of the cell and biological tissue transporting container A10 when the container body 3 is filled with a culture medium M1. In the present embodiment, the container body 3 is formed of a flexible material, and the tubular side wall portion 32 of the container body 3 is inserted into the opening 110 of the base member 1. Then, by screwing the internal thread 221 of the lid body 2 (the second tubular portion 22) onto the external thread 111 of the base member 1 (the first tubular portion 11), the flange 33 of the container body 3 is clamped between the peripheral edge portion of the opening 110 of the base member 1 (the upper end portion 113 of the first tubular portion 11) and the top plate portion 21 of the lid body 2, whereby the assembled state is maintained. With this configuration, the contents (the cultured cells and the culture medium) of the container body 3 are sealed in a liquid-tight manner. Accordingly, spilling of the contents due to vibrations and the like is prevented during cultured state-maintaining transportation. The external thread 111 of the base member 1 (the first tubular portion 11) and the internal thread 221 of the lid body 2 (the second tubular portion 22) constitute the lock of the present invention.

According to the present embodiment, by the cooperation of the base member 1, the lid body 2, and the container body 3, the contents of the container body 3 can be sealed duly in a liquid-tight manner. Besides, since the container body 3 is flexible, cells or a biological tissue contained in the container body 3 is less likely to be damaged when the cells or the biological tissue is unintentionally brought into direct contact with the container body 3. Therefore, the cell and biological tissue transporting container A10 provided with the container body 3 is suitable for cultured state-maintaining transportation.

In the present embodiment, the bottom wall portion 31 of the container body 3 is provided with the multiple protrusions 312, and the device 4 can be held by these protrusions 312. According to this configuration, for the device 4 applied to cell culture in a state of being set in a well plate, cultured state-maintaining transportation is possible while maintaining a liquid-tight state by replacing it from the well plate to the container body 3.

The bottom wall portion 31 of the container body 3 is provided with the gas permeable thin portion 311. Thus, the contents of the container body 3 remain in communication with the atmosphere outside the container body 3. Therefore, according to the present embodiment, it is possible to culture the contents of the container body 3 under ventilated conditions during the transportation.

The through hole 121 is formed in the bottom portion 12 of the base member 1. Also, the slit 112 is formed at the lower end of the first tubular portion 11. According to this configuration, even when the base member 1 in the form of a container is placed on a flat surface, the thin portion 311 of the container body 3 is not blocked by the base member 1. Therefore, this configuration is suitable for ensuring that the container body 3 is in communication with the atmosphere outside the container body 3.

The lid body 2 has the protruding portion 23 (the inner cylindrical portion 231 and the bottom plate portion 232), and the inner cylindrical portion 231 and the bottom plate portion 232 protrude toward the bottom wall portion 31 inside the side wall portion 32 of the container body 3. This configuration reduces the volume of a space for containing a culture medium and the like in the container body 3. Therefore, it is possible to reduce the amount of the culture medium M1 to be used in cultured state-maintaining transportation.

After the transportation of the cell and biological tissue transporting container A10, the lid body 2 can be detached from the base member 1 by loosening the threaded engagement portion between the base member 1 and the lid body 2, whereby the upper portion of the container body 3 can be opened. Therefore, unlike the case where an opening of a container is closed with an adhesive film or the like, it is possible to prevent a trouble that the contents of the container body 3 may spill out when detaching the lid body 2.

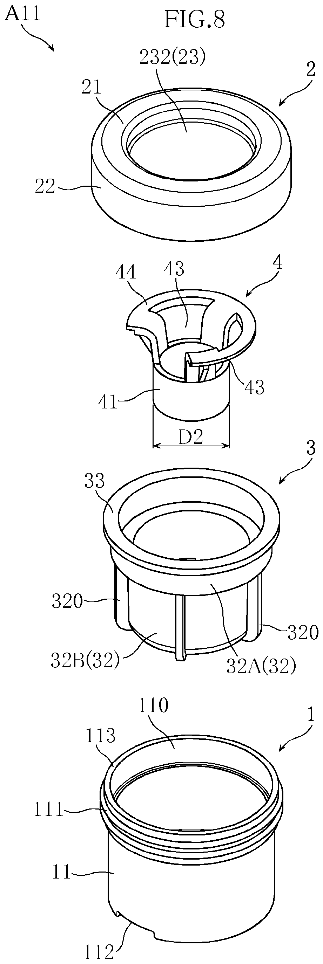

FIGS. 8 to 11 show a modified example of the above-described cell and biological tissue transporting container A10. The cell and biological tissue transporting container shown in these drawings is different from the above-described cell and biological tissue transporting container A10 mainly in the configuration of a container body. In FIG. 8 and the subsequent drawings, components that are identical or similar to those of the above embodiment are denoted by the same reference numerals as those used in the above embodiment, and the descriptions thereof may be omitted as appropriate.

In a cell and biological tissue transporting container A11 shown in FIGS. 8 to 11, a side wall portion 32 of a container body 3 has a large-diameter portion 32A and a small-diameter portion 32B. The large-diameter portion 32A is located closer to a flange 33, and opposes the inner surface of a portion where an external thread 111 is formed in a first tubular portion 11 of a base member 1. The small-diameter portion 32B is located closer to a bottom wall portion 31 and has a smaller radial dimension than the large-diameter portion 32A.

In the present modified example, multiple outer surface projections 320 are provided on the outer surface of the small-diameter portion 32B. The multiple outer surface projections 320 are disposed radially as viewed in the vertical direction. Each outer surface projection 320 can abut against the inner surface of the first tubular portion 11.

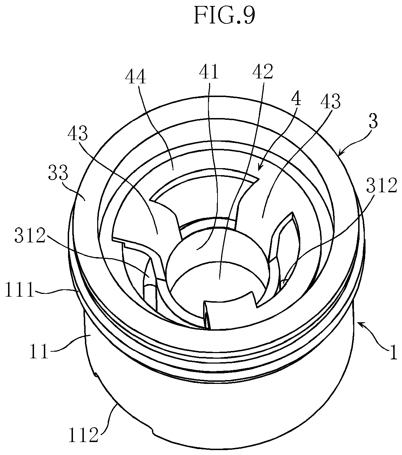

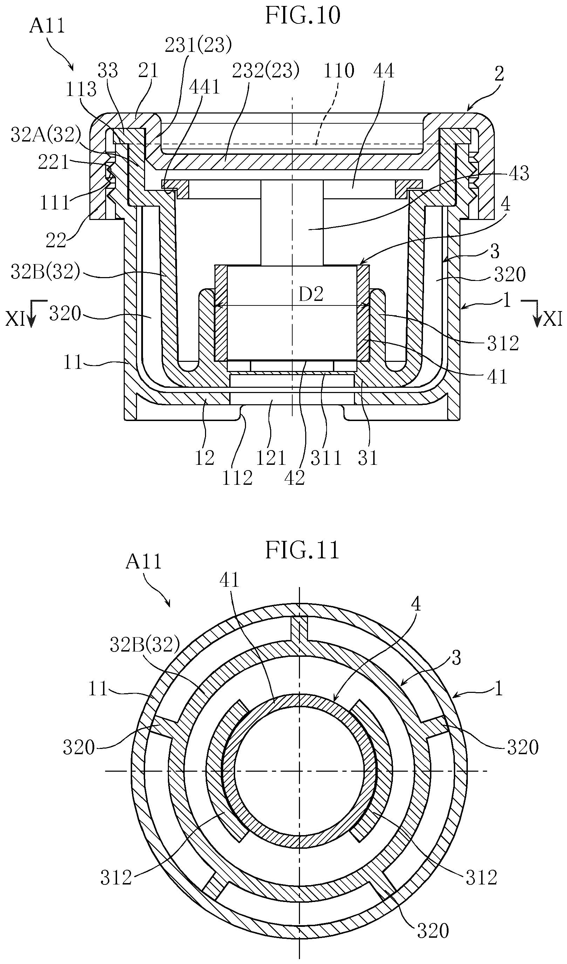

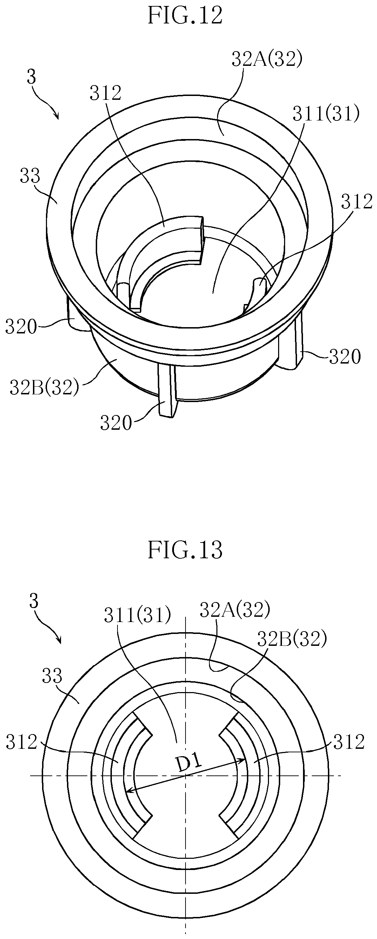

In the present modified example, the bottom wall portion 31 of the container body 3 is also provided with multiple protrusions 312. These protrusions 312 have the same configuration as the protrusions 312 in the cell and biological tissue transporting container A10. In the present modified example, the inner diameter dimension D1 (see FIG. 13) of the circle constituted by the multiple protrusions 312 in the container body 3 is also substantially the same as the outer diameter dimension D2 (see FIGS. 8 and 10) of the culture portion 41 (the cylindrical portion) or slightly smaller than the outer diameter dimension D2. Accordingly, by inserting the culture portion 41 inside the multiple protrusions 312, the device 4 is held in a predetermined orientation inside the container body 3, as shown in FIGS. 9 and 10. As shown in FIG. 10, in the state where the device 4 is held, the upper end of the small-diameter portion 32B of the container body 3 is in a recess 441 provided on the lower side of the outer periphery of a ring-shaped portion 44 in the device 4, and thus the upper end is in close proximity to the ring-shaped portion 44.

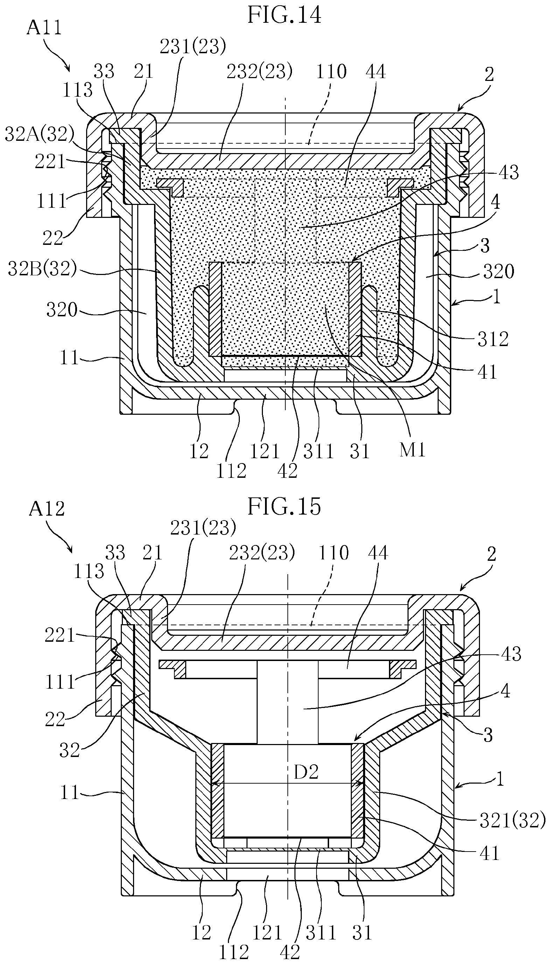

FIG. 14 shows the assembled state of the cell and biological tissue transporting container A11 when the container body 3 is filled with a culture medium M1. The container body 3 is formed of a flexible material, and the tubular side wall portion 32 of the container body 3 is inserted into the opening 110 of the base member 1. Then, by screwing the internal thread 221 of the lid body 2 (the second tubular portion 22) onto the external thread 111 of the base member 1 (the first tubular portion 11), the flange 33 of the container body 3 is clamped between the peripheral edge portion of the opening 110 of the base member 1 (the upper end portion 113 of the first tubular portion 11) and the top plate portion 21 of the lid body 2, whereby the assembled state is maintained. With this configuration, the contents (cultured cells and a culture medium) of the container body 3 are sealed in a liquid-tight manner. Accordingly, spilling of the contents due to vibrations and the like is prevented during cultured state-maintaining transportation.

According to the present modified example, by the cooperation of the base member 1, the lid body 2, and the container body 3, the contents of the container body 3 can be sealed duly in a liquid-tight manner. Besides, since the container body 3 is flexible, cells or a biological tissue contained in the container body 3 is less likely to be damaged when the cells or the biological tissue is unintentionally brought into direct contact with the container body 3. Therefore, the cell and biological tissue transporting container A11 provided with the container body 3 is suitable for cultured state-maintaining transportation.

The bottom wall portion 31 of the container body 3 is provided with the multiple protrusions 312, and the device 4 can be held by these protrusions 312. According to this configuration, for the device 4 applied to cell culture in a state of being set in a well plate, cultured state-maintaining transportation is possible while maintaining a liquid-tight state by replacing it from the well plate to the container body 3.

The side wall portion 32 of the container body 3 has the large-diameter portion 32A and the small-diameter portion 32B. The small-diameter portion 32B is located closer to the bottom wall portion 31, and the culture portion 41 of the device 4 is housed in this small-diameter portion 32B. According to such a configuration, the radial dimension of the small-diameter portion 32B can be set so as to be closely analogous to the radial dimension of a well plate in which the device 4 is set. Therefore, during the transportation of the device 4, it is possible to provide an environment similar to that when the device 4 is housed inside the well plate. Furthermore, according to the configuration in which the small-diameter portion 32B is provided, the volume of a space for containing a culture medium and the like in the container body 3 is reduced. Therefore, it is possible to reduce the amount of the culture medium M1 to be used in cultured state-maintaining transportation.

On the outer surface of the small-diameter portion 32B, the multiple outer surface projections 320 are provided radially. According to such a configuration, it is possible to perform positioning of the container body 3 in the radial direction with respect to the base member 1.

The bottom wall portion 31 of the container body 3 is provided with the gas permeable thin portion 311. Thus, the contents of the container body 3 remain in communication with the atmosphere outside the container body 3. Therefore, according to the present modified example, it is possible to culture the contents of the container body 3 under ventilated conditions during the transportation.

A through hole 121 is formed in a bottom portion 12 of the base member 1. Also, a slit 112 is formed at the lower end of the first tubular portion 11. According to this configuration, even when the base member 1 in the form of a container is placed on a flat surface, the thin portion 311 of the container body 3 is not blocked by the base member 1. Therefore, this configuration is suitable for ensuring that the container body 3 is in communication with the atmosphere outside the container body 3.

The lid body 2 has the protruding portion 23 (the inner cylindrical portion 231 and the bottom plate portion 232), and the inner cylindrical portion 231 and the bottom plate portion 232 protrude toward the bottom wall portion 31 inside the side wall portion 32 of the container body 3. This configuration reduces the volume of a space for containing a culture medium and the like in the container body 3. Therefore, it is possible to reduce the amount of the culture medium M1 to be used in cultured state-maintaining transportation.

After the transportation of the cell and biological tissue transporting container A11, the lid body 2 can be detached from the base member 1 by loosening the threaded engagement portion between the base member 1 and the lid body 2, whereby the upper portion of the container body 3 can be opened. Therefore, unlike the case where an opening of a container is closed with an adhesive film or the like, it is possible to prevent a trouble that the contents of the container body 3 may spill out when detaching the lid body 2.

FIGS. 15 to 17 show other modified examples of the above-described cell and biological tissue transporting container A10. Cell and biological tissue transporting containers shown in these drawings are each different from the above-described cell and biological tissue transporting container A10 only in the configuration of a container body.

In a cell and biological tissue transporting container A12 shown in FIG. 15, a container body 3 has an overhanging portion 321 instead of the protrusions 312 in the above embodiment. The overhanging portion 321 is a portion of a side wall portion 32 on the lower end side, which overhangs radially inward such that this portion has a smaller diameter than a remaining portion. The inner diameter dimension of the overhanging portion 321 is substantially the same as the outer diameter dimension D2 of a culture portion 41 (a cylindrical portion) or slightly smaller than the outer diameter dimension D2. Accordingly, by inserting a culture portion 41 inside the overhanging portion 321, a device 4 is held in a predetermined orientation inside the container body 3.

In the cell and biological tissue transporting container A12 of the present modified example, by screwing an internal thread 221 of a lid body 2 (a second tubular portion 22) onto an external thread 111 of a base member 1 (a first tubular portion 11), a flange 33 of the container body 3 is clamped between the peripheral edge portion of an opening 110 of the base member 1 (an upper end portion 113 of the first tubular portion 11) and a top plate portion 21 of the lid body 2, whereby the assembled state is maintained. With this configuration, during cultured state-maintaining transportation, the contents (cultured cells and a culture medium) of the container body 3 are sealed in a liquid-tight manner and spilling of the contents due to vibrations and the like is prevented.

In addition to the above, the cell and biological tissue transporting container A12 of the present modified example exhibits the same functions and effects as the above-described cell and biological tissue transporting container A10.

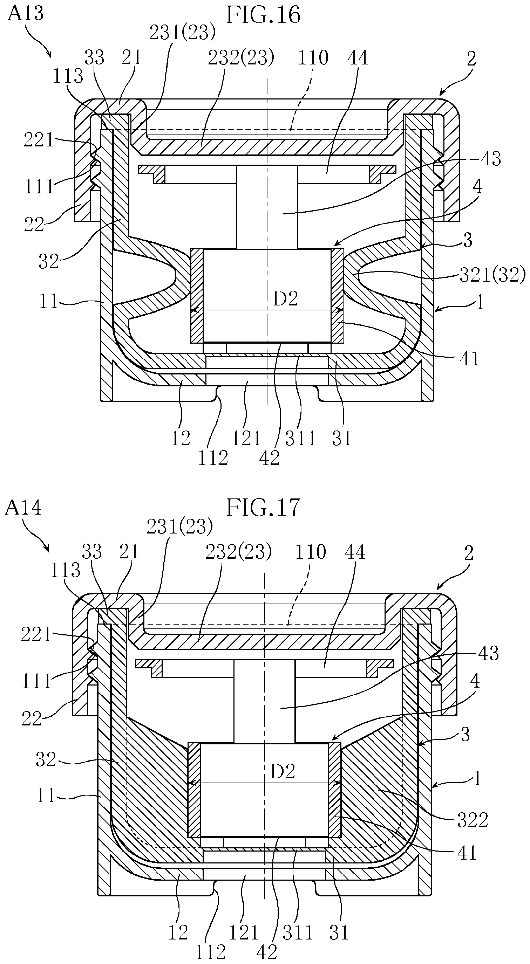

In a cell and biological tissue transporting container A13 shown in FIG. 16, a container body 3 has an overhanging portion 321 instead of the protrusions 312 in the above-described cell and biological tissue transporting container A10. The overhanging portion 321 is a central portion of a side wall portion 32 in the vertical direction, which overhangs radially inward such that this portion has a smaller diameter than a remaining portion. The inner diameter dimension of the overhanging portion 321 is substantially the same as the outer diameter dimension D2 of a culture portion 41 (a cylindrical portion) or slightly smaller than the outer diameter dimension D2. Accordingly, by inserting a culture portion 41 inside the overhanging portion 321, a device 4 is held in a predetermined orientation inside the container body 3.

In the cell and biological tissue transporting container A13 of the present modified example, by screwing an internal thread 221 of a lid body 2 (a second tubular portion 22) onto an external thread 111 of a base member 1 (a first tubular portion 11), a flange 33 of the container body 3 is clamped between the peripheral edge portion of an opening 110 of the base member 1 (an upper end portion 113 of the first tubular portion 11) and a top plate portion 21 of the lid body 2, whereby the assembled state is maintained. With this configuration, during cultured state-maintaining transportation, the contents (cultured cells and a culture medium) of the container body 3 are sealed in a liquid-tight manner and spilling of the contents due to vibrations and the like is prevented.

In addition to the above, the cell and biological tissue transporting container A13 of the present modified example exhibits the same functions and effects as the above-described cell and biological tissue transporting container A10.

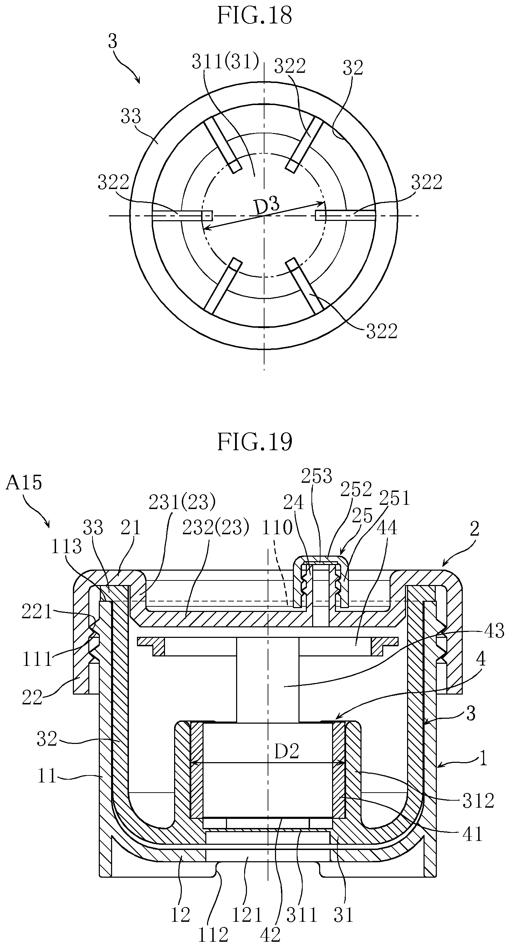

In a cell and biological tissue transporting container A14 shown in FIG. 17, a container body 3 has multiple ribs 322 instead of the protrusions 312 in the above-described cell and biological tissue transporting container A10. The multiple ribs 322 each extend radially inward from a side wall portion 32, and as shown in FIG. 18, the multiple ribs 322 are disposed radially as viewed in the vertical direction. The diameter D3 (see FIG. 18) of a virtual circle formed by the respective inner ends of the multiple ribs 322 is substantially the same as the outer diameter dimension D2 of a culture portion 41 (a cylindrical portion) or slightly smaller than the outer diameter dimension D2. Accordingly, by inserting the culture portion 41 inside the multiple ribs 322, the device 4 is held in a predetermined orientation inside the container body 3.

In the cell and biological tissue transporting container A14 of the present modified example, by screwing an internal thread 221 of a lid body 2 (a second tubular portion 22) onto an external thread 111 of a base member 1 (a first tubular portion 11), a flange 33 of the container body 3 is clamped between the peripheral edge portion of an opening 110 of the base member 1 (an upper end portion 113 of the first tubular portion 11) and a top plate portion 21 of the lid body 2, whereby the assembled state is maintained. With this configuration, during cultured state-maintaining transportation, the contents (cultured cells and a culture medium) of the container body 3 are sealed in a liquid-tight manner and spilling of the contents due to vibrations and the like is prevented.

In addition to the above, the cell and biological tissue transporting container A14 of the present modified example exhibits the same functions and effects as the above-described cell and biological tissue transporting container A10.

FIG. 19 shows still another modified example of the above-described cell and biological tissue transporting container A10. A cell and biological tissue transporting container A15 shown in this drawing is different from the above-described cell and biological tissue transporting container A10 only in the configuration of a lid body.

In the cell and biological tissue transporting container A15 shown in FIG. 19, a lid body 2 has a small-diameter tubular nozzle portion 24 and a cap 25. In the present modified example, the nozzle portion 24 is provided in a protruding portion 23 (a bottom plate portion 232) and is in communication with the internal space of a container body 3. The nozzle portion 24 is generally cylindrical, and an external thread is formed on the outer peripheral surface of the nozzle portion 24. The cap 25 is configured such that, for example, the upper end of a tubular portion 251 that extends generally in a cylindrical shape is closed by a top plate 252. An internal thread is formed on the inner peripheral surface of the tubular portion 251, and the top plate 252 is provided with a sheet-like sealing member 253 made of rubber, for example. By screwing the internal thread of the cap 25 onto the external thread of the nozzle portion 24, the tip of the nozzle portion 24 is closed.

In the cell and biological tissue transporting container A15 of the present modified example, by screwing an internal thread 221 of the lid body 2 (a second tubular portion 22) onto an external thread 111 of a base member 1 (a first tubular portion 11), a flange 33 of the container body 3 is clamped between the peripheral edge portion of an opening 110 of the base member 1 (an upper end portion 113 of the first tubular portion 11) and a top plate portion 21 of the lid body 2, whereby the assembled state is maintained. With this configuration, during cultured state-maintaining transportation, the contents (cultured cells and a culture medium) of the container body 3 are sealed in a liquid-tight manner and spilling of the contents due to vibrations and the like is prevented.

In the present modified example, the lid body 2 has the small-diameter tubular nozzle portion 24 and the cap 25 that can close the nozzle portion 24. According to such a configuration, after the lid body 2 is attached to the base member 1, it is possible to refill the internal space of the container body 3 with a culture medium (liquid) via the nozzle portion 24. This allows the internal space of the container body 3 to be filled with the culture medium (liquid) with the amount of air (gas) remaining in the internal space of the container body 3 being reduced as much as possible. Consequently, it is possible to prevent the occurrence of air entrainment at the time of screwing the lid body 2 and also to restrict shaking of the culture medium during cultured state-maintaining transportation. Also, at the time of screwing the lid body 2, a pressure may be applied to cultured cells contained in the container body 3. However, by opening the cap 25 once and then closing the cap 25, it is possible to relieve the pressure.

In addition to the above, the cell and biological tissue transporting container A15 of the present modified example exhibits the same functions and effects as the above-described cell and biological tissue transporting container A10.

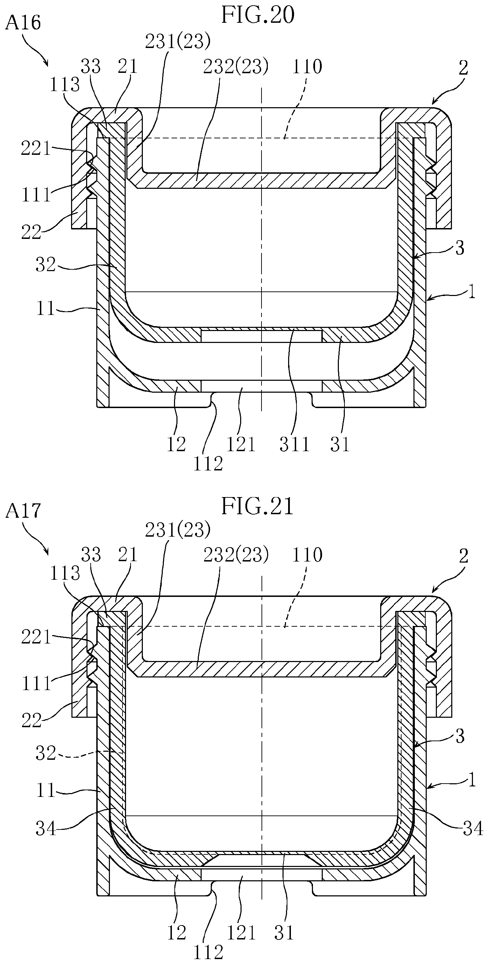

FIGS. 20 and 21 show still other modified examples of the above-described cell and biological tissue transporting container A10. Cell and biological tissue transporting containers shown in these drawings are each different from the above-described cell and biological tissue transporting container A10 in the configuration of a container body. Besides, these cell and biological tissue transporting containers are each configured such that a device is not housed in a container body.

In a cell and biological tissue transporting container A16 shown in FIG. 20, a container body 3 does not have the protrusions 312 provided in the above-described cell and biological tissue transporting container A10. Also, at the time of cultured state-maintaining transportation, cultured cells or a biological tissue is placed inside the container body 3 directly, and the container body 3 is filled with a culture medium.

In the cell and biological tissue transporting container A16 of the present modified example, by screwing an internal thread 221 of a lid body 2 (a second tubular portion 22) onto an external thread 111 of a base member 1 (a first tubular portion 11), a flange 33 of the container body 3 is clamped between the peripheral edge portion of an opening 110 of the base member 1 (an upper end portion 113 of the first tubular portion 11) and a top plate portion 21 of the lid body 2, whereby the assembled state is maintained. With this configuration, during cultured state-maintaining transportation, the contents (cultured cells and a culture medium) of the container body 3 are sealed in a liquid-tight manner and spilling of the contents due to vibrations and the like is prevented.

A bottom wall portion 31 of the container body 3 is provided with a gas permeable thin portion 311. Thus, the contents of the container body 3 remain in communication with the atmosphere outside the container body 3. Therefore, according to the present modified example, it is possible to culture the contents of the container body 3 under ventilated conditions during the transportation.

A through hole 121 is formed in a bottom portion 12 of the base member 1. Also, a slit 112 is formed at the lower end of the first tubular portion 11. According to this configuration, even when the base member 1 in the form of a container is placed on a flat surface, the thin portion 311 of the container body 3 is not blocked by the base member 1. Therefore, this configuration is suitable for ensuring that the container body 3 is in communication with the atmosphere outside the container body 3.

The lid body 2 has a protruding portion 23 (an inner cylindrical portion 231 and a bottom plate portion 232), and the inner cylindrical portion 231 and the bottom plate portion 232 protrude toward the bottom wall portion 31 inside a side wall portion 32 of the container body 3. This configuration reduces the volume of a space for containing a culture medium and the like in the container body 3. Therefore, it is possible to reduce the amount of the culture medium to be used in cultured state-maintaining transportation.

After the transportation of the cell and biological tissue transporting container A16, the lid body 2 can be detached from the base member 1 by loosening the threaded engagement portion between the base member 1 and the lid body 2, whereby the upper portion of the container body 3 can be opened. Therefore, unlike the case where an opening of a container is closed with an adhesive film or the like, it is possible to prevent a trouble that the contents of the container body 3 may spill out when detaching the lid body 2.

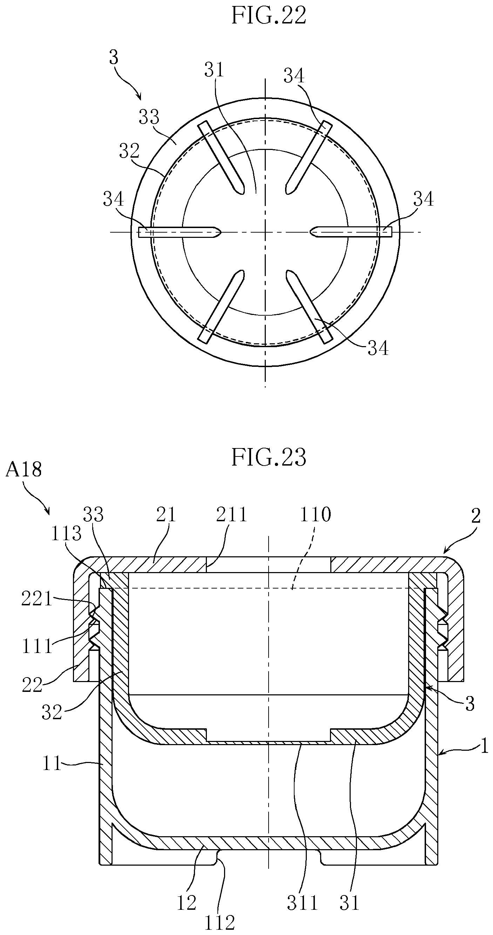

In a cell and biological tissue transporting container A17 shown in FIG. 21, a container body 3 does not have the protrusions 312 provided in the above-described cell and biological tissue transporting container A10. In the present modified example, an entire bottom wall portion 31 and an entire side wall portion 32 of the container body 3 are relatively thin and gas permeable. Multiple reinforcing ribs 34 are provided at appropriate positions in the bottom wall portion 31 and the outer surface of the side wall portion 32. As shown in FIG. 22, these reinforcing ribs 34 are provided radially as viewed in the vertical direction. In the cell and biological tissue transporting container A17 shown in FIG. 21, at the time of cultured state-maintaining transportation, cultured cells or a biological tissue is placed inside the container body 3 directly, and the container body 3 is filled with a culture medium.

In the cell and biological tissue transporting container A17 of the present modified example, by screwing an internal thread 221 of a lid body 2 (a second tubular portion 22) onto an external thread 111 of a base member 1 (a first tubular portion 11), a flange 33 of the container body 3 is clamped between the peripheral edge portion of an opening 110 of the base member 1 (an upper end portion 113 of the first tubular portion 11) and a top plate portion 21 of the lid body 2, whereby the assembled state is maintained. With this configuration, during cultured state-maintaining transportation, the contents (cultured cells and a culture medium) of the container body 3 are sealed in a liquid-tight manner and spilling of the contents due to vibrations and the like is prevented.

The entire bottom wall portion 31 and the entire side wall portion 32 of the container body 3 are gas permeable thin portions. Thus, the contents of the container body 3 remain in communication with the atmosphere outside the container body 3. Therefore, according to the present modified example, it is possible to culture the contents of the container body 3 under ventilated conditions during the transportation.

In addition to the above, the cell and biological tissue transporting container A17 of the present modified example exhibits the same functions and effects as the above-described cell and biological tissue transporting container A16.

In each of the above-described cell and biological tissue transporting containers A10 to A17, the through hole 121 is formed in the bottom portion 12 of the base member 1. However, when it is not necessary to ensure the ventilated conditions in view of the transportation conditions and the like, a through hole need not be formed in the bottom portion 12 of the base member 1 and the bottom portion 12 may be closed.

FIG. 23 shows still another modified example of the above-described cell and biological tissue transporting container A10. A cell and biological tissue transporting container A18 shown in this drawing is different from the above-described cell and biological tissue transporting container A10 in the configurations of a base member, a lid body, and a container body.

In the cell and biological tissue transporting container A18 shown in FIG. 23, a bottom portion 12 of a base member 1 does not have the through hole 121 provided in the above-described cell and biological tissue transporting container A10. Therefore, the base member 1 is in the form of a cylindrical container with the bottom portion 12 being closed. A container body 3 does not have the protrusions 312 provided in the above-described cell and biological tissue transporting container A10. Also, a device is not housed in the container body 3. On the other hand, a lid body 2 is configured such that a top plate portion 21 is flat, and in the top plate portion 21, a through hole 211 that passes through the top plate portion 21 in the thickness direction is formed.

In the cell and biological tissue transporting container A18 of the present modified example, at the time of cultured state-maintaining transportation, cultured cells or a biological tissue is placed inside the base member 1 directly, and a space between the base member 1 and the container body 3 is filled with a culture medium, unlike the case of the above embodiment.

In the cell and biological tissue transporting container A18 of the present modified example, by screwing an internal thread 221 of the lid body 2 (a second tubular portion 22) onto an external thread 111 of the base member 1 (a first tubular portion 11), a flange 33 of the container body 3 is clamped between the peripheral edge portion of an opening 110 of the base member 1 (an upper end portion 113 of the first tubular portion 11) and the top plate portion 21 of the lid body 2, whereby the assembled state is maintained. With this configuration, during cultured state-maintaining transportation, the contents (cultured cells and a culture medium) of the base member 1 are sealed in a liquid-tight manner and spilling of the contents due to vibrations and the like is prevented.

A bottom wall portion 31 of the container body 3 is provided with a gas permeable thin portion 311. Thus, the contents of the base member 1 remain in communication with the atmosphere in the internal space of the container body 3. Therefore, according to the present modified example, the contents of the base member 1 can be cultured under ventilated conditions during the transportation.

The through hole 211 is formed in the top plate portion 21 of the lid body 2. According to such a configuration, the internal space of the container body 3 is in communication with the outside of the lid body 2 via the through hole 211. Thus, this configuration is suitable for ensuring that the contents filling the space between the base member 1 and the container body 3 are in communication with the outside atmosphere.

After the transportation of the cell and biological tissue transporting container A18, the lid body 2 and the container body 3 can be detached one by one from the base member 1 by loosening the threaded engagement portion between the base member 1 and the lid body 2, whereby the upper portion of the base member 1 can be opened. Therefore, unlike the case where an opening of a container is closed with an adhesive film or the like, it is possible to prevent a trouble that the contents of the base member 1 may spill out when detaching the lid body 2.

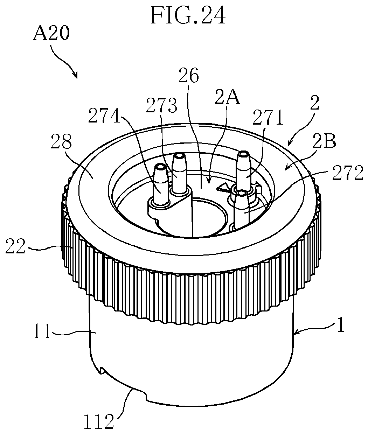

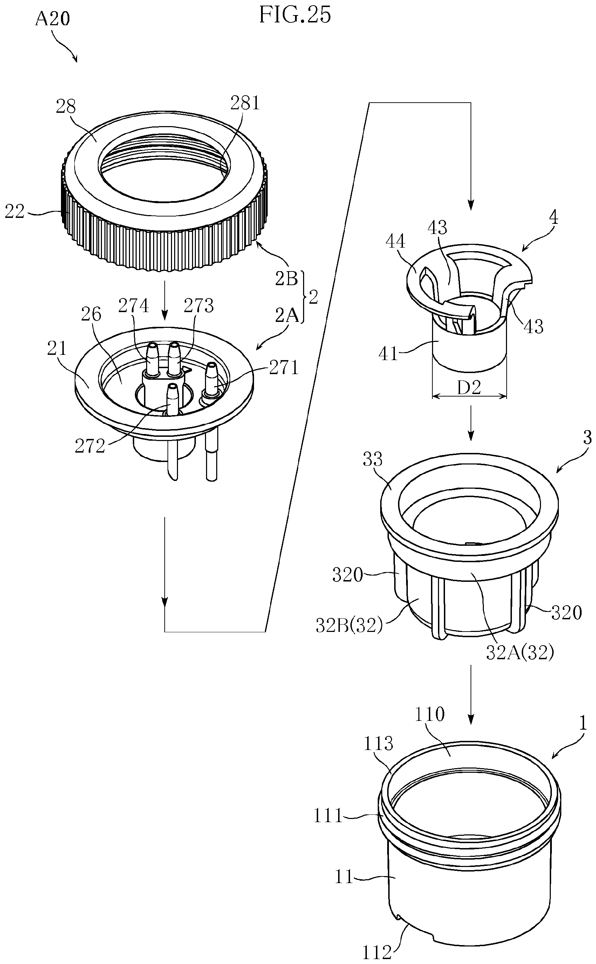

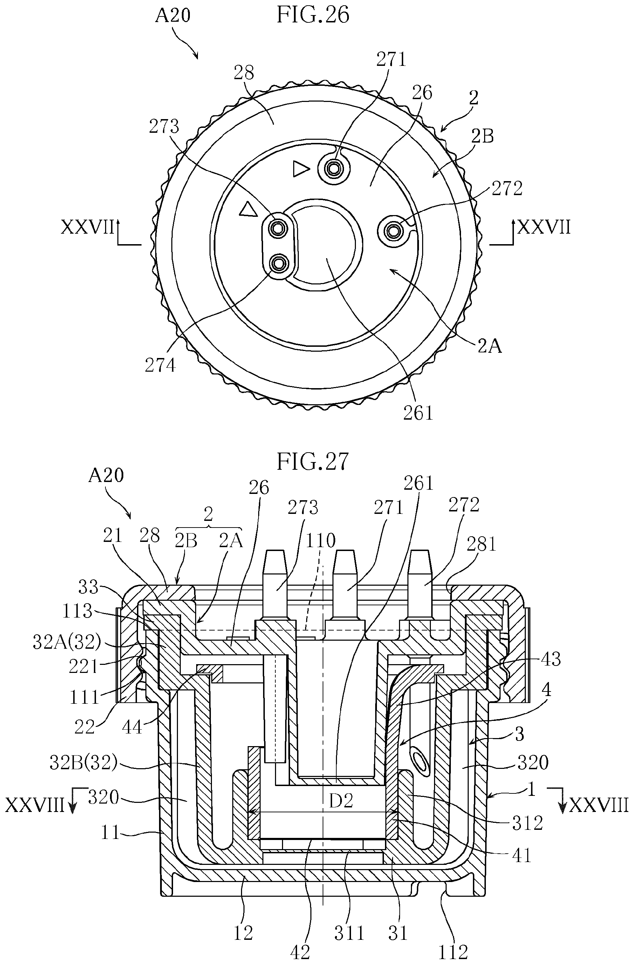

FIGS. 24 to 28 show the second embodiment of the cell and biological tissue transporting container according to the present invention. A cell and biological tissue transporting container A20 of the present embodiment includes a base member 1, a lid body 2, and a container body 3. FIGS. 24, 26, and 27 show an assembled state in which the base member 1, the lid body 2, and the container body 3 are combined with one another, and this will be described more specifically below. In the present embodiment, as can be seen in FIG. 27, a device 4 is held by the container body 3. FIG. 25 is a perspective view showing the components of the cell and biological tissue transporting container A20 in a disassembled state.

According to the cell and biological tissue transporting container A20 of the present embodiment, processes from cell culture to transportation can be carried out using only the cell and biological tissue transporting container A20. That is, while cell culture using the device 4 is performed using a culture container different from the cell and biological tissue transporting container in the above embodiment, cell culture using a device 4 is also performed using the cell and biological tissue transporting container A20 in the present embodiment. The cell and biological tissue transporting container A20 is a sealed container adapted so that cell culture can be performed therein using an automated culture apparatus without opening a lid.

The cell and biological tissue transporting container A20 of the present embodiment is greatly different from the cell and biological tissue transporting containers A10 and A11 of the above-described embodiments in the configuration of a lid body 2. The lid body 2 includes an inner lid 2A and an outer lid 2B. The inner lid 2A has a top plate portion 21, a stepped portion 26, and multiple (four in the present embodiment) ports 271, 272, 273, and 274.

The inner lid 2A is a component for sealing a container body 3. In the present embodiment, the top plate portion 21 of the inner lid 2A is annular. On the top plate portion 21, a presser portion 28 (the outer lid 2B) to be described below is stacked in the thickness direction of the top plate portion 21. The stepped portion 26 is located inside the top plate portion 21. The stepped portion 26 is accommodated inside a side wall portion 32 (a large-diameter portion 32A) of the container body 3 and protrudes toward a bottom wall portion 31 of the container body 3 in the vertical direction. Furthermore, in the present embodiment, the stepped portion 26 is provided with a bottom plate portion 261 having a recessed shape. The bottom plate portion 261 is recessed downward to be located closer to the bottom wall portion 31 of the container body 3 and is located at a central portion in the radial direction.

In the present embodiment, the four ports 271, 272, 273, and 274 (first port, second port, third port, and fourth port) are provided in the stepped portion 26. These ports 271, 272, 273, and 274 are provided for supplying a culture medium and the like to the inside of the container body 3 or for discharging the culture medium in the container body 3 to the outside. The ports 271, 272, 273, and 274 are each configured such that an upper end thereof is open to the outside and a lower end thereof is open to the internal space of the container body 3. To the upper ends of the respective ports 271, 272, 273, and 274, tubes (not shown) are connected when necessary. The two ports 271 and 272 are provided near the side wall portion 32 (the small-diameter portion 32B) of the container body 3. The remaining two ports 273 and 274 are provided farther inward in the radial direction of the top plate portion 21 than the ports 271 and 272. Of the ports 273 and 274, the port 274 has its lower end at a relatively high position and is used for supplying, e.g., a cell suspension, a culture medium, and a predetermined gas to the inside of the device 4. Of the ports 273 and 274, the port 273 has its lower end at a relatively low position and is used for discharging the culture medium and the like inside the device 4. Of the ports 271 and 272, the port 272 has its lower end at a relatively high position and is used for supplying, e.g., a culture medium and the like to the inside of the container body 3. Of the ports 271 and 272, the port 271 has its lower end at a relatively low position and is used for discharging the culture medium and the like inside the container body 3.

The inner lid 2A is formed of a translucent or transparent plastic material, for example. In the inner lid 2A, at least the bottom plate portion 261 is transparent. The bottom plate portion 261 is relatively thin and has a smooth surface. Accordingly, the bottom plate portion 261 is a transparent portion through which an internal space can be observed visually from the outside.

The outer lid 2B has the presser portion 28 and a second tubular portion 22, and presses the top plate portion 21 toward a flange 33 of the container body 3. The presser portion 28 is stacked on the top plate portion 21 as viewed in the thickness direction of the top plate portion 21. In the present embodiment, the presser portion 28 is annular, and an opening hole 281 is formed inside the presser portion 28. The second tubular portion 22 is connected to the outer peripheral edge of the presser portion 28. The second tubular portion 22 has the same configuration as the second tubular portion 22 in the above-described cell and biological tissue transporting container A11.

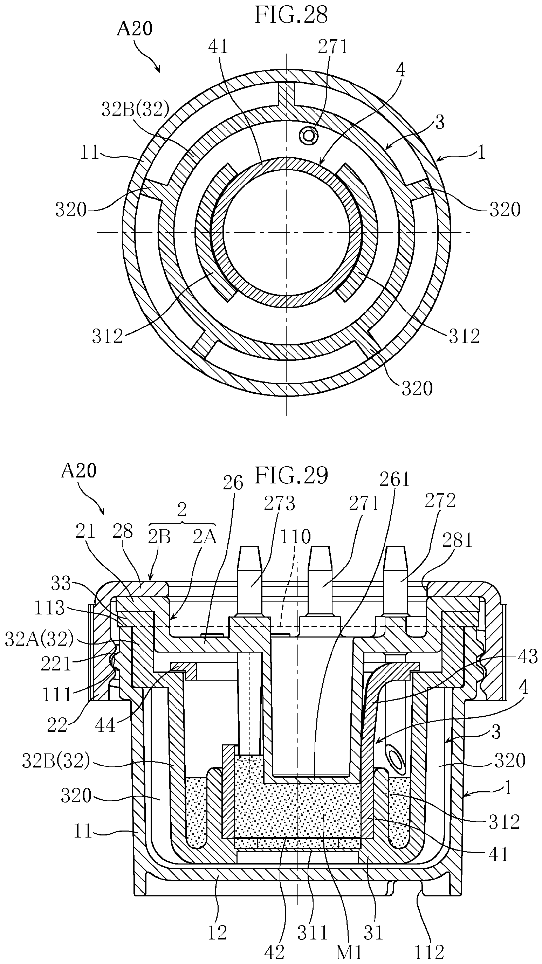

The container body 3 in the present embodiment has the same configuration as the container body 3 in the above-described cell and biological tissue transporting container A11. That is, the container body 3 has the bottom wall portion 31, the side wall portion 32 that rises from the peripheral edge of the bottom wall portion 31, and the flange 33. The side wall portion 32 of the container body 3 has the large-diameter portion 32A and the small-diameter portion 32B. Multiple outer surface projections 320 are provided on the outer surface of the small-diameter portion 32B. As shown in FIG. 28, the multiple outer surface projections 320 are disposed radially as viewed in the vertical direction. Each outer surface projection 320 can abut against the inner surface of a first tubular portion 11. As shown in FIG. 27, the bottom wall portion 31 has a thin portion 311 provided in a central portion thereof.

In the present embodiment, the base member 1 is a sealed container without a through hole in the bottom portion 12, and therefore, the thin portion 311 need not be gas permeable. On the other hand, the thin portion 311 has a smooth surface and is transparent. For example, in the case of a material like silicone rubber, it is important to make the material thin in order to ensure transparency. The bottom wall portion 31 is provided with multiple protrusions 312. In the present embodiment, the inner diameter dimension of the circle constituted by the multiple protrusions 312 in the container body 3 is also substantially the same as the outer diameter dimension D2 (see FIGS. 25 and 27) of a culture portion 41 (a cylindrical portion) or slightly smaller than the outer diameter dimension D2. Accordingly, by inserting the culture portion 41 inside the multiple protrusions 312, the device 4 is held in a predetermined orientation inside the container body 3, as shown in FIG. 27. As shown in FIG. 27, in the state where the device 4 is held, the upper end of the small-diameter portion 32B of the container body 3 is in a recess 441 provided on the lower side of the outer periphery of a ring-shaped portion 44 in the device 4, and thus the upper end is in close proximity to the ring-shaped portion 44.

As can be understood from FIGS. 25 to 27, among the four ports 271 to 274, the two ports 273 and 274 are provided at positions to be inside the culture portion 41. These ports 273 and 274 are provided at positions displaced from the center of the bottom plate portion 261 so as to avoid a central portion of the bottom plate portion 261 and thus are located at positions near the inner peripheral surface of the culture portion 41. The remaining two ports 271 and 272 are provided at positions to be in a region between the culture portion 41 and the side wall portion 32 (the small-diameter portion 32B) of the container body 3.

In the present embodiment, the bottom portion 12 of the base member 1 does not have the through hole 121 provided in the above-described cell and biological tissue transporting containers A10 and A11. Therefore, the base member 1 is in the form of a cylindrical container with the bottom portion 12 being closed.

Next, usage and functions of the cell and biological tissue transporting container A20 will be described.

The cell and biological tissue transporting container A20 of the present embodiment is used for performing cell culture in this container A20 and then transporting the cells while maintaining the cultured state (cultured state-maintaining transportation).

In the present embodiment, the lid body 2 includes the inner lid 2A and the outer lid 2B, the inner lid 2A is provided with the top plate portion 21 and the ports 271, 272, 273, and 274, and the outer lid 2B is provided with the presser portion 28 and the second tubular portion 22 (an internal thread 221) . According to such a configuration, at the time of attaching the lid body 2 to the base member 1, the inner lid 2A provided with the ports is set at a predetermined position, and thereafter, the outer lid 2B is screwed, during which the inner lid 2A (the ports 271, 272, 273, and 274) does not move. Therefore, the lid body 2 provided with the ports can be assembled to a predetermined position in the sealed container properly.

At the time of cell culture, a cell suspension, a culture medium, and a predetermined gas (e.g., CO₂ gas or the like) is supplied into the device 4 via the port 274, for example. To the tube (not shown) connected to the port 274, containers filled with the cell suspension, the culture medium, and the predetermined atmosphere gas are connected, for example, and the substance desired to be supplied is selected as appropriate from these containers by the switching of a valve or the like and is supplied into the device 4 via the port 274. To the container body 3, a culture medium is supplied via the port 272, for example.

FIG. 29 shows an example of a state where cell culture is performed using the cell and biological tissue transporting container A20. During the cell culture, for example, the internal space of the container body 3 is maintained at a predetermined temperature by the temperature adjustment function of the automated culture apparatus. Furthermore, the internal space of the container body 3 is maintained in a predetermined gas atmosphere, and the culture medium in the container body 3 and the culture medium in the device 4 are discharged as appropriate via the port 271 and the port 273, respectively, for replacement.

In the cell culture performed using the cell and biological tissue transporting container A20, a cell sheet is produced by, for example, adding highly transparent collagen gel, which is not shown in the drawings, onto a thin film 42 of the device 4 and seeding cells on the collagen gel. In the present embodiment, the bottom plate portion 261 is transparent. Accordingly, from above the cell and biological tissue transporting container A20, the state in the culture portion 41 of the device 4 can be observed through the transparent bottom plate portion 261.

In the present embodiment, the thin portion 311 of the container body 3 is transparent. When the base member 1 is transparent, also from below the cell and biological tissue transporting container A20, the state in the culture portion 41 of the device 4 can be observed through the transparent bottom portion 12 (the base member 1) and the thin portion 311. With such a configuration, regardless of whether a light source is placed above or below the container, irradiation light can be transmitted, and therefore, the cells can be observed using various types of microscopes including an optical microscope and a phase-contrast microscope.

The ports 273 and 274 provided in the inner lid 2A are at the displaced positions avoiding the central portion of the inner lid 2A. According to such a configuration, at the time of observing the state in the culture portion 41 of the device 4 from above the cell and biological tissue transporting container A20 through the transparent bottom plate portion 261, interference of the ports 273 and 274 is avoided.

In the present embodiment, the bottom plate portion 261 (the transparent portion at the center) of the inner lid 2A has a recessed shape that is recessed downward. By disposing the bottom plate portion 261 at a low position as described above, a culture solution in the device 4 is in contact with the surface of the bottom plate portion 261. Accordingly, the surface of the culture solution becomes flat owing to a water glass effect, whereby diffuse reflection of irradiation light is suppressed, and this is expected to bring about an effect that focusing of a microscope is performed easily.

In the present embodiment, the bottom wall portion 31 of the flexible container body 3 is provided with the multiple protrusions 312, and the device 4 can be held by these protrusions 312. Such a configuration is suitable for microscopic observation because the device 4 in which cell culture is performed is fixed to a predetermined position accurately.

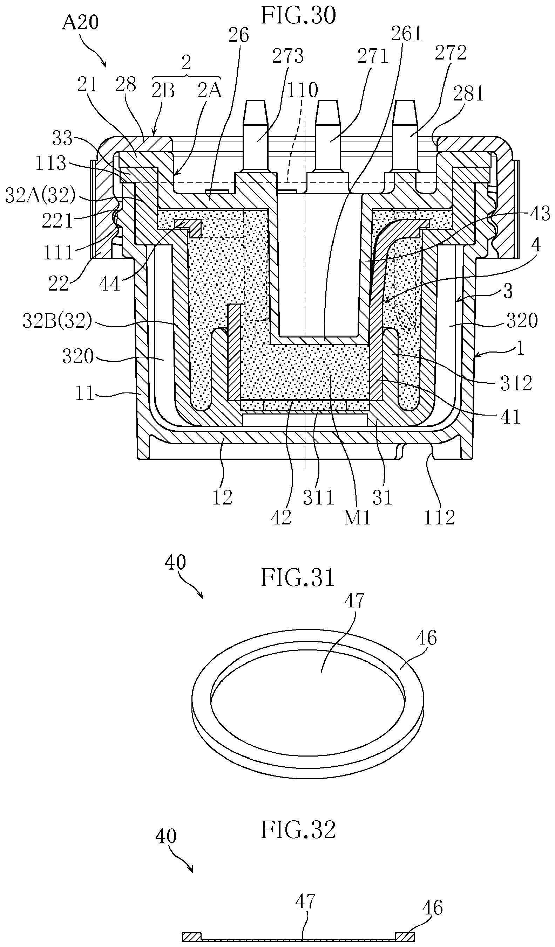

FIG. 30 shows the assembled state of the cell and biological tissue transporting container A20 when the container body 3 is filled with a culture medium M1. The container body 3 is formed of a flexible material, and the tubular side wall portion 32 of the container body 3 is inserted into an opening 110 of the base member 1. Then, by screwing the internal thread 221 of the lid body 2 (the second tubular portion 22) onto the external thread 111 of the base member 1 (the first tubular portion 11), the flange 33 of the container body 3 is clamped between the peripheral edge portion of the opening 110 of the base member 1 (the upper end portion 113 of the first tubular portion 11) and the top plate portion 21 of the lid body 2, whereby the assembled state is maintained. With this configuration, the contents (cultured cells and the culture medium) of the container body 3 are sealed in a liquid-tight manner. Accordingly, spilling of the contents due to vibrations and the like is prevented during cultured state-maintaining transportation. Furthermore, by filling the container body 3 completely with the culture medium without leaving a space in the container body 3, shaking of the culture medium due to vibrations and the like is prevented, and the cells immersed in the culture medium can be transported safely while maintaining the cultured state of the cells. At the time of transportation of the cell and biological tissue transporting container A20, the tubes (not shown) connected to the ports 271, 272, 273, and 274 are sealed by appropriate means such as heat sealing.

According to the present embodiment, by the cooperation of the base member 1, the lid body 2, and the container body 3, the contents of the container body 3 can be sealed duly in a liquid-tight manner.

In the present embodiment, the bottom wall portion 31 of the flexible container body 3 is provided with the multiple protrusions 312, and the device 4 can be held by these protrusions 312. With this configuration, for the device 4 in which cell culture is performed, vibrations during transportation can be relieved by the flexibility of the protrusions 312, and the device 4 can be subjected to cultured state-maintaining transportation in the state of being held by the container body 3 while maintaining a liquid-tight state.

The side wall portion 32 of the container body 3 has the large-diameter portion 32A and the small-diameter portion 32B. The small-diameter portion 32B is located closer to the bottom wall portion 31, and the culture portion 41 of the device 4 is housed in this small-diameter portion 32B. According to the configuration in which the small-diameter portion 32B is provided, the volume of a space for containing a culture medium and the like in the container body 3 is reduced. Therefore, it is possible to reduce the amount of the culture medium M1 to be used in cultured state-maintaining transportation.

On the outer surface of the small-diameter portion 32B, the multiple outer surface projections 320 are provided radially. According to such a configuration, it is possible to perform positioning of the container body 3 in the radial direction with respect to the base member 1.

The lid body 2 (the inner lid 2A) has the stepped portion 26, and the stepped portion 26 protrudes toward the bottom wall portion 31 inside the side wall portion 32 of the container body 3. This configuration reduces the volume of a space for containing a culture medium and the like in the container body 3. Therefore, it is possible to reduce the amount of the culture medium M1 to be used in cultured state-maintaining transportation.

After the transportation of the cell and biological tissue transporting container A20, the lid body 2 can be detached from the base member 1 by loosening the threaded engagement portion between the base member 1 and the lid body 2, whereby the upper portion of the container body 3 can be opened.

Regarding the above-described cell and biological tissue transporting container A20, the above description is directed to a case where, as a member on which cultured cells are to be placed, the device 4 is set in the container body 3. However, the shape of the member on which cultured cells are to be placed is not limited thereto. For example, instead of the above-described device 4, a support 40 shown in FIGS. 31 and 32 may be used. The support 40 has a frame 46 and a thin film 47. The frame 46 is in a ring shape, and the thin film 47 is formed so as to cover a space inside this frame 46. The thin film 47 is a portion on which cultured cells (a cell sheet) are to be placed, and the cultured cells on the thin film 47 are immersed in a culture medium. Alternatively, the member on which the cultured cells are to be placed need not be used. For example, a cell suspension may be contained in a region surrounded by the container body 3 and the lid body 2, and a biological tissue or the like may be placed in a region surrounded by the protrusions 312 protruding from the bottom wall portion 31 of the container body 3. The shape of each protrusion 312 is not limited to an arc shape.

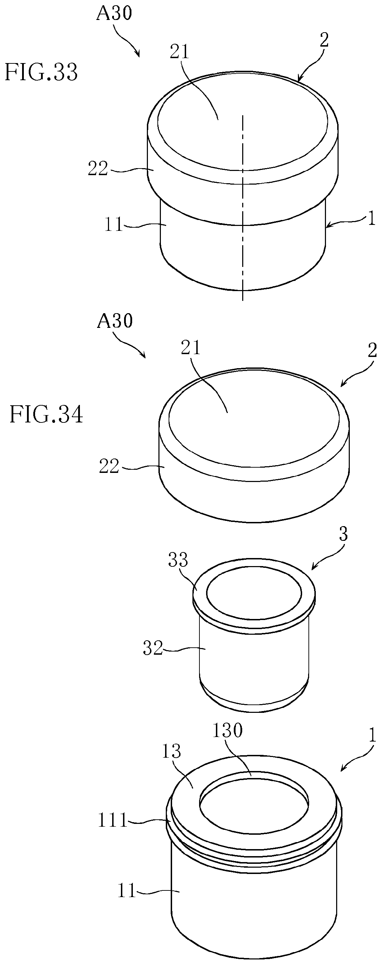

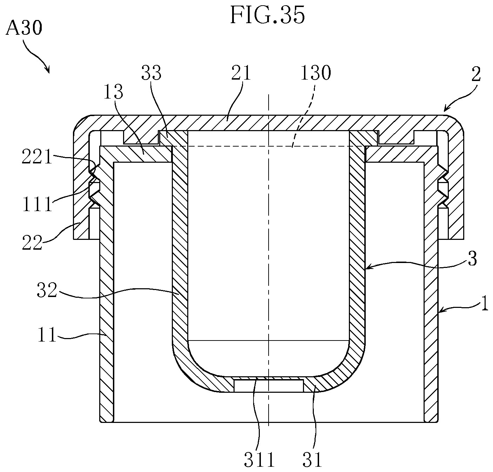

FIGS. 33 to 35 show the third embodiment of the cell and biological tissue transporting container according to the present invention. A cell and biological tissue transporting container A30 of the present embodiment includes a base member 1, a lid body 2, and a container body 3. FIGS. 33 and 35 show an assembled state in which the base member 1, the lid body 2, and the container body 3 are combined with one another. FIG. 34 is a perspective view showing the components of the cell and biological tissue transporting container A30 in a disassembled state.

In the present embodiment, the base member 1 includes a first tubular portion 11 and an upper plate13. The first tubular portion 11 is generally cylindrical, and an external thread 111 is formed on an upper outer peripheral surface of the first tubular portion 11. The upper plate 13 is in an annular shape that is connected to an upper end of the first tubular portion 11 and extends radially inward from the upper end. An opening 130 is formed at an inner edge of the upper plate 13, and the opening 130 is located at the upper end of the base member 1.

The lid body 2 has a top plate portion 21 and a second tubular portion 22, and is a component for sealing the container body 3. A flange 33 (to be described below) of the container body 3 is clamped between the top plate portion 21 and the upper plate 13 of the base member 1. The second tubular portion 22 extends from the peripheral edge of the top plate portion 21 in the thickness direction of the top plate portion 21 (downward in the drawings), and is generally cylindrical. An internal thread 221 is formed on the inner peripheral surface of the second tubular portion 22, and the internal thread 221 is threadedly engageable with the external thread 111 of the base member 1 (the first tubular portion 11).

The container body 3 is formed of a flexible and resilient material as in the above-described first embodiment, and the container body 3 has a bottom wall portion 31, a side wall portion 32, and a flange 33. The bottom wall portion 31 has a gas permeable thin portion 311 provided in a central portion thereof. On the other hand, in the cell and biological tissue transporting container A30, the container body 3 does not have the protrusions 312 provided in the above-described cell and biological tissue transporting container A10. At the time of cultured state-maintaining transportation, cultured cells or a biological tissue is placed inside the container body 3 directly, and the container body 3 is filled with a culture medium.

In the assembled state shown in FIG. 35, the outer surface of the side wall portion 32 opposes the inner surface of the first tubular portion 11 in the base member 1. In the assembled state, the side wall portion 32 is inserted into the opening 130 of the base member 1, and the side wall portion 32 and the bottom wall portion 31 are located inside the first tubular portion 11 of the base member 1.

Next, functions of the cell and biological tissue transporting container A30 will be described.

In the present embodiment, the container body 3 is formed of a flexible material, and the tubular side wall portion 32 of the container body 3 is inserted into the opening 130 of the base member 1. Then, by screwing the internal thread 221 of the lid body 2 (the second tubular portion 22) onto the external thread 111 of the base member 1 (the first tubular portion 11), the flange 33 of the container body 3 is clamped between the peripheral edge portion (the upper plate 13) of the opening 130 of the base member 1 and the top plate portion 21 of the lid body 2, whereby the assembled state is maintained. With this configuration, the contents (cultured cells and a culture medium) of the container body 3 are sealed in a liquid-tight manner. Accordingly, spilling of the contents due to vibrations and the like is prevented during cultured state-maintaining transportation.

According to the present embodiment, by the cooperation of the base member 1, the lid body 2, and the container body 3, the contents of the container body 3 can be sealed duly in a liquid-tight manner. Besides, since the container body 3 is flexible, cells or a biological tissue contained in the container body 3 is less likely to be damaged when the cells or the biological tissue is unintentionally brought into direct contact with the container body 3. Therefore, the cell and biological tissue transporting container A30 provided with the container body 3 is suitable for cultured state-maintaining transportation.

The bottom wall portion 31 of the container body 3 is provided with the gas permeable thin portion 311. Thus, the contents of the container body 3 remain in communication with the atmosphere outside the container body 3. Therefore, according to the present embodiment, it is possible to culture the contents of the container body 3 under ventilated conditions during the transportation.

After the transportation of the cell and biological tissue transporting container A30, the lid body 2 can be detached from the base member 1 by loosening the threaded engagement portion between the base member 1 and the lid body 2, whereby the upper portion of the container body 3 can be opened. Therefore, unlike the case where an opening of a container is closed with an adhesive film or the like, it is possible to prevent a trouble that the contents of the container body 3 may spill out when detaching the lid body 2.

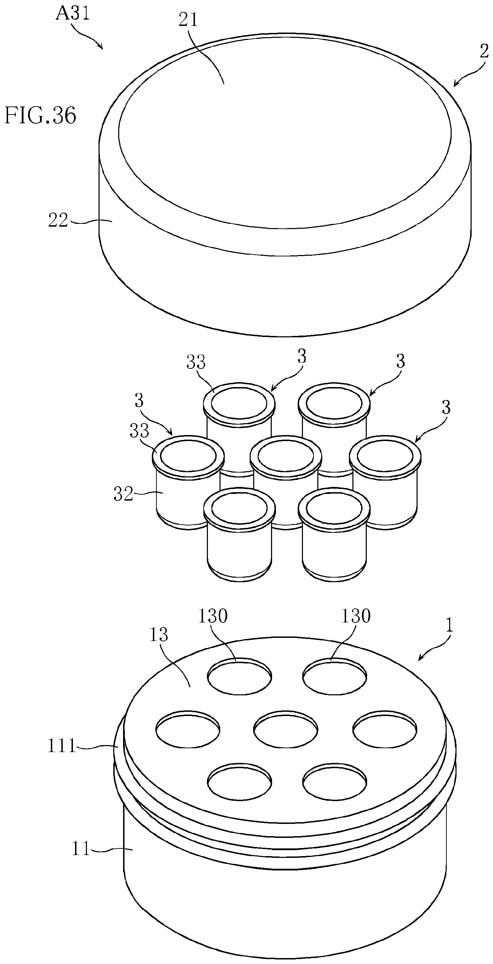

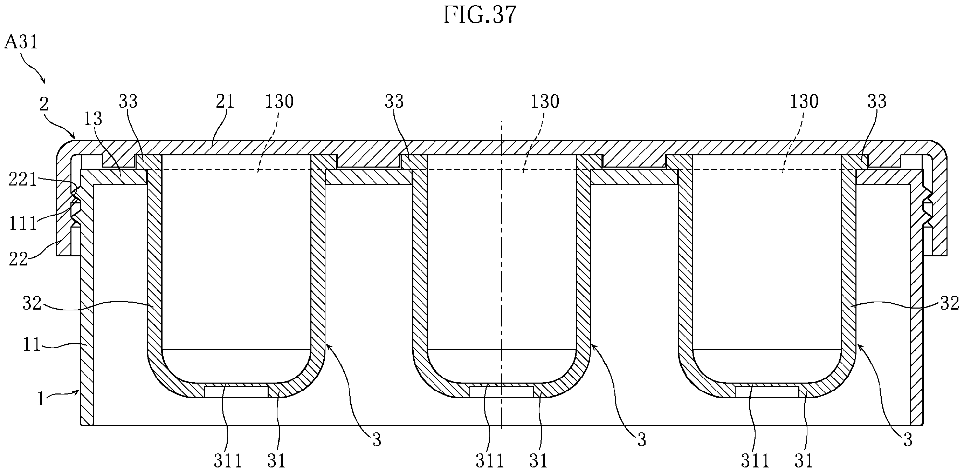

FIGS. 36 and 37 show a modified example of the cell and biological tissue transporting container A30 according to the third embodiment. A cell and biological tissue transporting container A31 shown in these drawings is different from the above-described cell and biological tissue transporting container A30 in the configurations of a base member and a lid body.

As shown in FIGS. 36 and 37, a base member 1 includes a first tubular portion 11 and an upper plate13. The first tubular portion 11 is generally cylindrical, and an external thread 111 is formed on an upper outer peripheral surface of the first tubular portion 11. The first tubular portion 11 in the present modified example has a larger radial dimension than the first tubular portion 11 in the third embodiment. The upper plate 13 is in a circular shape that closes the upper end of the first tubular portion 11. The upper plate 13 has multiple (seven in the present modified example) openings 130 that are formed so as to be apart from each other. These openings 130 are located at the upper end of the base member 1.

A lid body 2 has a top plate portion 21 and a second tubular portion 22, and is a component for sealing container bodies 3. Flanges 33 (to be described below) of the container bodies 3 are clamped between the top plate portion 21 and the upper plate 13 of the base member 1. The second tubular portion 22 extends from the peripheral edge of the top plate portion 21 in the thickness direction of the top plate portion 21 (downward in the drawings), and is generally cylindrical. An internal thread 221 is formed on the inner peripheral surface of the second tubular portion 22, and the internal thread 221 is threadedly engageable with the external thread 111 of the base member 1 (the first tubular portion 11).

The cell and biological tissue transporting container A31 of the present modified example includes multiple (seven in the present modified example) container bodies 3. Each of these container bodies 3 has the same configuration as the container body 3 in the above-described cell and biological tissue transporting container A30. In the assembled state shown in FIG. 37, side wall portions 32 of the respective container bodies 3 are inserted into the openings 130 of the base member 1, and the respective side wall portions 32 and the respective bottom wall portions 31 are located inside the first tubular portion 11 of the base member 1. At the time of cultured state-maintaining transportation, cultured cells or a biological tissue is placed inside each container body 3 directly, and each container body 3 is filled with a culture medium.

Next, functions of the cell and biological tissue transporting container A31 will be described.

In the cell and biological tissue transporting container A31 of the present modified example, the respective container bodies 3 are formed of a flexible material, and the tubular side wall portions 32 of the respective container bodies 3 are inserted into the openings 130 of the base member 1. Then, by screwing the internal thread 221 of the lid body 2 (the second tubular portion 22) onto the external thread 111 of the base member 1 (the first tubular portion 11), the flanges 33 of the respective container bodies 3 are clamped between the peripheral edge portions (the upper plate 13) of the openings 130 in the base member 1 and the top plate portion 21 of the lid body 2, whereby the assembled state is maintained. With this configuration, the contents (cultured cells and a culture medium) of the container body 3 are sealed in a liquid-tight manner. Accordingly, spilling of the contents due to vibrations and the like is prevented during cultured state-maintaining transportation.

In the present modified example, by providing the multiple container bodies 3, it becomes possible to transport multiple types of cultured cells or biological tissues at the same time.

In addition to the above, the cell and biological tissue transporting container A31 of the present modified example exhibits the same functions and effects as the above-described cell and biological tissue transporting container A30.

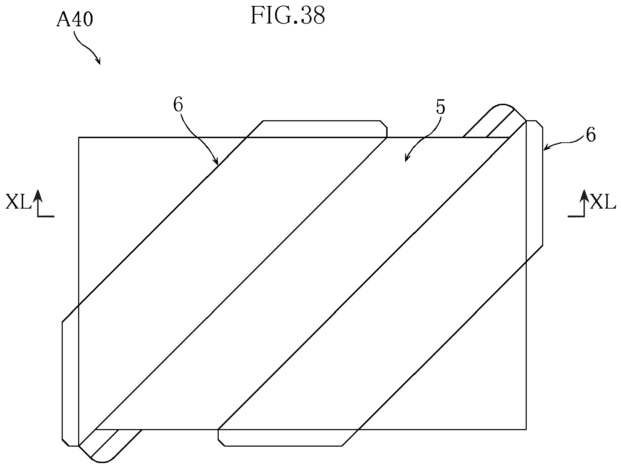

FIGS. 38 to 40 show a cell and biological tissue transporting container of a reference example as a variation of the present invention.

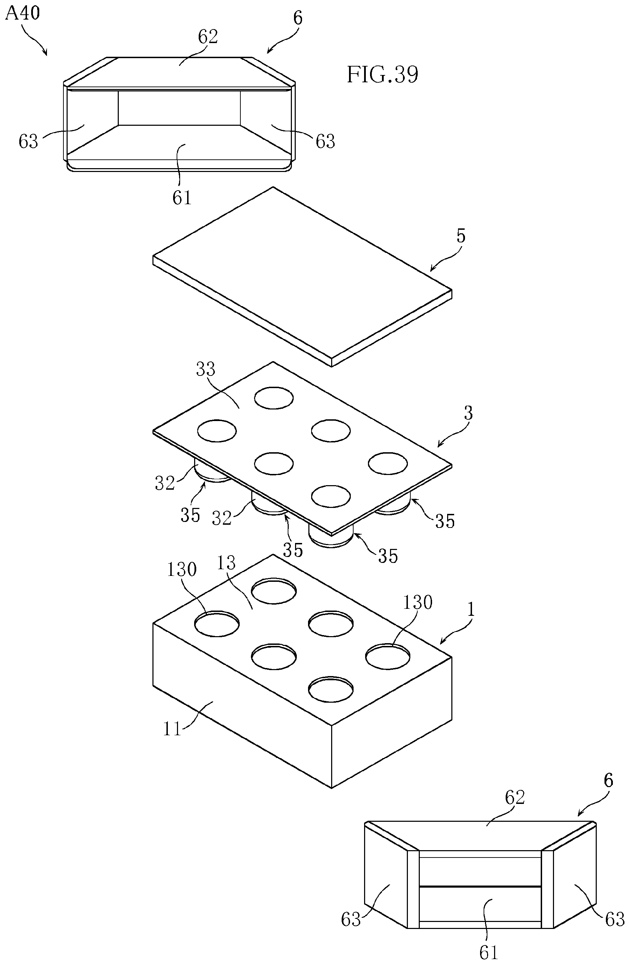

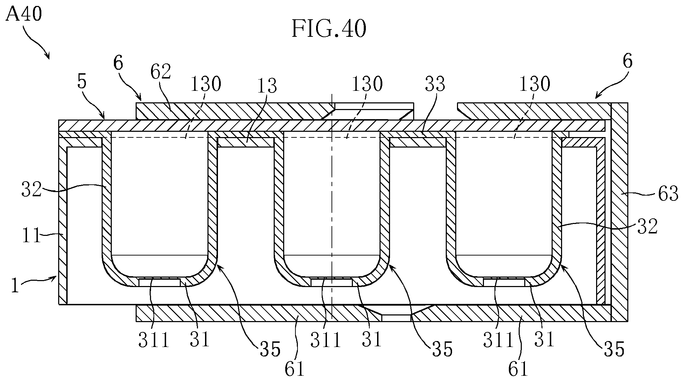

The cell and biological tissue transporting container A40 of the present reference example includes a base member 1, a container body 3, a presser plate 5, and holders 6. FIGS. 38 and 40 show an assembled state in which the base member 1, the container body 3, and the pressure plate 5 are stacked. FIG. 39 is a perspective view showing the components of the cell and biological tissue transporting container A40 in a disassembled state.

As shown in FIGS. 39 and 40, the base member 1 includes a first tubular portion 11 and an upper plate 13. The first tubular portion 11 is generally in a rectangular tubular shape. The upper plate 13 is in a rectangular shape that closes the upper end of the first tubular portion 11. The upper plate 13 has multiple (six in the present reference example) openings 130 that are formed so as to be apart from each other. These openings 130 are located at the upper end of the base member 1.

The container body 3 is a component for containing cultured cells and a culture medium. The container body 3 is formed of a flexible and resilient material. Also, the container body 3 is preferably self-adhesive. Examples of the material of the container body 3 include silicone rubber, natural rubber, urethane rubber, and elastomer resin. Among them, silicone rubber is preferable. In light of the contact between the container body 3 and the contents (the cultured cells and the culture medium) to be described specifically below, it is more preferable that the material of the container body 3 is medical grade silicone rubber, which is free of cytotoxicity and has biocompatibility. Regarding the hardness of the container body 3, it is preferable that the container body 3 has a rubber hardness of about 20 to 40 degrees.

The container body 3 is a rubber molded product, for example, and as shown in FIG. 40, the container body 3 includes bottom wall portions 31, side wall portions 32 that rise from the peripheral edges of the bottom wall portions 31, and a flange 33. Each bottom wall portion 31 is substantially circular in a plan view. Each side wall portion 32 is generally cylindrical. In the present reference example, the container body has six bottom wall portions 31 and six side wall portions 32. Each bottom wall portion 31 and the side wall portion 32 connected thereto constitute a vessel 35. In the present reference example, the container body 3 has six vessels 35.

The flange 33 is generally in a rectangular shape, and extends outward from the upper edges of the respective side wall portions 32 so as to surround the side wall portions 32. The flange 33 has an appropriate thickness and appropriate elastic resilience against loads applied from the top and the bottom of the flange 33. The flange 33 has a thickness of about 1 to 3 mm, for example. In the assembled state shown in FIG. 40, the respective side wall portions 32 are inserted into the openings 130 of the base member 1, and the respective vessels 35 (the side wall portions 32 and the bottom wall portions 31) are located inside the first tubular portion 11 of the base member 1. At the time of cultured state-maintaining transportation, cultured cells or a biological tissue is placed inside each vessel 35 directly, and each vessel 35 is filled with a culture medium.

As shown in FIG. 40, in the present reference example, each bottom wall portion 31 has a thin portion 311 provided in a central portion thereof. As clear from FIG. 40, the thin portion 311 has a smaller thickness than other portions. The thin portion 311 has a thickness of about 0.2 to 0.3 mm, for example. The thin portion 311 is gas permeable.

The presser plate 5 is placed on the flange 33 in the container body 3. The presser plate 5 is in a rectangular plate shape, and the external size thereof is substantially the same as the external size of the flange 33. The presser plate 5 is formed of hard synthetic resin such as polyvinyl chloride, polypropylene, polyethylene, and polystyrene, for example.

The holders 6 hold the base member 1, the container body 3, and the presser plate 5 in the state where they are stacked (the assembled state) . As shown in FIG. 39, each holder 6 has a flat bottom plate 61, a ceiling plate 62 that extends parallel to this bottom plate 61, and a pair of side plates 63, and these plate members together form a loop shape. Both ends of each of the pair of side plates 63 are connected to the bottom plate 61 and the ceiling plate 62, respectively. The cell and biological tissue transporting container A40 of the present reference example is provided with two holders 6.

The distance from the upper surface of the bottom plate 61 to the lower surface of the ceiling plate 62 is slightly shorter than the height dimension of an assembly formed by stacking the base member 1, the container body 3, and the presser plate 5 without undue force. When the base member 1, the container body 3, and the presser plate 5 are held altogether by gripping the assembly with a hand while applying pressure from above and below the assembly, the flange 33 in the container body 3 is compressed, whereby the height dimension of the assembly is reduced. As a result, it becomes possible to insert the assembly between the bottom plate 61 and the ceiling plate 62 of each holder 6. Then, when the hand is released, the height dimension is caused to increase owing to the elastic resilience of the flange 33. However, the bottom plate 61 and the ceiling plate 62 restrict the movement in a direction to increase the height dimension. At this time, the base member 1, the container body 3, and the presser plate 5 are pressed from above and below by the bottom plates 61 and the ceiling plates 62 in the assembled state where they are stacked on one another, whereby they are held integrally by the holders 6.

Next, usage and functions of the cell and biological tissue transporting container A40 will be described.

The cell and biological tissue transporting container A40 is used for containing cultured cells or a biological tissue in the container body 3 (each vessel 35) together with a culture medium and transporting the cultured cells or the biological tissue while maintaining the cultured state (cultured state-maintaining transportation). The cells, biological tissue, and culture medium to be contained in each vessel 35 are not particularly limited.

For example, a cell preparation for use in regenerative medicine can be contained in the form of a suspension or a cell sheet overlaid on the device. When transporting the cell suspension or cell sheet in a non-frozen state, in order to prevent the culture medium from being shaken to damage the cells, it is necessary to fill each vessel 35 completely with the culture medium to restrict the movement of the culture medium.

In the present reference example, the container body 3 is formed of a flexible material, and the respective tubular side wall portions 32 of the container body 3 are inserted into the openings 130 of the base member 1. Then, by holding the base member 1, the container body 3, and the presser plate 5 integrally by the holders 6 in a state where they are stacked together, the flange 33 of the container body 3 is clamped between the peripheral edge portion (the upper plate 13) of the openings 130 of the base member 1 and the presser plate 5, whereby the assembled state is maintained. With this configuration, the contents (cultured cells and a culture medium) of the container body 3 (each vessel 35) are sealed in a liquid-tight manner. Accordingly, spilling of the contents due to vibrations and the like is prevented during cultured state-maintaining transportation.

According to the present reference example, by cooperation of the base member 1, the container body 3, the presser plate 5, and the holders 6, the contents of the container body 3 can be sealed duly in a liquid-tight manner. Besides, since the container body 3 is flexible, cells or a biological tissue contained in the container body 3 is less likely to be damaged when the cells or the biological tissue is unintentionally brought into direct contact with the container body 3. Therefore, the cell and biological tissue transporting container A40 provided with the container body 3 is suitable for cultured state-maintaining transportation.

The bottom wall portion 31 of the container body 3 (vessels 35) is provided with the gas permeable thin portions 311. Thus, the contents of the container body 3 remain in communication with the atmosphere outside the container body 3. Therefore, according to the present reference example, it is possible to culture the contents of the container body 3 under ventilated conditions during the transportation.

After the transportation of the cell and biological tissue transporting container A40, by pulling out the base member 1, the container body 3, and the presser plate 5 in the assembled state from the holders 6, the base member 1, the container body 3, and the presser plate 5 can be disassembled easily. That is, the base member 1, the container body 3, and the presser plate 5 in the assembled state are merely stacked on one another. Accordingly, the presser plate 5 can be detached smoothly to open the upper part of the container body 3. Therefore, unlike the case where an opening of a container is closed with an adhesive film or the like, it is possible to prevent a trouble that the contents of the container body 3 may spill out when opening the upper part of the container body 3.

While specific embodiments of the present invention have been described above, the present invention is by no means limited thereto, and various changes and modifications may be made without departing from the spirit of the present invention. Various changes and modifications in design may be made freely to specific configuration of respective portions of the cell and biological tissue transporting container according to the present invention.

Although the above embodiments have been described with reference to the examples where the lock is a threaded fastening mechanism (the external thread 111 of the first tubular portion 11 and the internal thread 221 of the second tubular portion 22), the present invention is not limited thereto. The lock may have any configuration as long as it can prevent relative movement between the base member and the lid body. For example, the following configurations may be employed as the lock: a configuration in which protrusions are provided in one and the other one of the first tubular portion and the second tubular portion, and by mutual engagement of these protrusions, the state where the relative movement between the base member and the lid body is prevented is maintained; or a configuration in which a recess is formed in one of the first tubular portion and the second tubular portion and a protrusion is provided in the other one of the first tubular portion and the second tubular portion, and by fitting the protrusion into the recess, the state where the relative movement between the base member and the lid body is prevented is maintained. Furthermore, as another example of the configuration of the lock, it is also possible to employ a hinge mechanism using pivotable locking members.

## Claims

1. A cell and biological tissue transporting container comprising:
a base member including an opening at an upper end and a first tubular portion extending vertically;
a lid body including a top plate portion to close the opening, and a second tubular portion that extends from an outer peripheral side of this top plate portion in a thickness direction of the top plate portion and fits onto the first tubular portion of the base member; and
a container body formed separately from the base member using a flexible material, the container body including a bottom wall portion, a tubular side wall portion that rises from a peripheral edge of this bottom wall portion and is inserted into the opening, and a flange that extends outward from an upper edge of this side wall portion,
wherein the base member and the lid body are provided with a lock that prevents relative movement between the base member and the lid body with the flange of the container body being clamped between a peripheral edge portion of the opening of the base member and the top plate portion of the lid body.

2. The cell and biological tissue transporting container according to claim 1, wherein
the side wall portion of the container body includes a large-diameter portion that is located closer to the flange and a small-diameter portion that is located closer to the bottom wall portion and has a smaller radial dimension than the large-diameter portion.

3. The cell and biological tissue transporting container according to claim 2, wherein
an outer surface of the small-diameter portion is provided with a plurality of outer surface projections that are disposed radially as viewed in a vertical direction and each of which is configured to abut against an inner surface of the first tubular portion of the base member.

4. The cell and biological tissue transporting container according to claim 1, wherein
an outer surface of the side wall portion of the container body abuts against an inner surface of the first tubular portion of the base member while opposing the inner surface.

5. The cell and biological tissue transporting container according to any one of claims 1 to 4, wherein
the lock includes an external thread that is provided in one of the first tubular portion and the second tubular portion and an internal thread that is provided in the other one of the first tubular portion and the second tubular portion and is threadedly engageable with the external thread.

6. The cell and biological tissue transporting container according to any one of claims 1 to 5, wherein
the flange of the container body is constituted by a thick portion that is relatively thick, and
at least one of the bottom wall portion and the side wall portion of the container body includes a thin portion that is relatively thin and gas permeable.

7. The cell and biological tissue transporting container according to claim 6, wherein
the base member includes a bottom portion that is connected to a lower end of the first tubular portion or a vicinity thereof and extends inwardly of the first tubular portion as viewed in a vertical direction,
the bottom portion of the base member extends inwardly of the first tubular portion from a position displaced upward from the lower end of the first tubular portion and has a through hole that passes through the base member in a thickness direction, and
a slit is formed at the lower end of the first tubular portion.

8. The cell and biological tissue transporting container according to claim 6, wherein
the base member includes a bottom portion that is connected to a lower end of the first tubular portion or a vicinity thereof and extends inwardly of the first tubular portion as viewed in a vertical direction,
the bottom portion of the base member closes an internal space of the first tubular portion, and
the top plate portion of the lid body has a through hole that passes through the top plate portion in a thickness direction.

9. The cell and biological tissue transporting container according to any one of claims 1 to 5, wherein
the flange of the container body is constituted by a thick portion that is relatively thick, and
the bottom wall portion of the container body includes a thin portion that is relatively thin and transparent.

10. The cell and biological tissue transporting container according to claim 9, wherein
the base member includes a bottom portion that is connected to a lower end of the first tubular portion or a vicinity thereof and closes an internal space of the first tubular portion.

11. The cell and biological tissue transporting container according to any one of claims 1 to 10, wherein
the lid body includes a protruding portion that is accommodated inside the side wall portion of the container body and protrudes toward the bottom wall portion of the container body.

12. The cell and biological tissue transporting container according to any one of claims 1 to 7, wherein
the lid body includes a small-diameter tubular nozzle portion that protrudes upward, and a cap to close a tip of the nozzle portion.

13. The cell and biological tissue transporting container according to any one of claims 1 to 7, wherein
the lid body includes : an inner lid including the top plate portion; and an outer lid including a presser portion that is stacked on the top plate portion as viewed in a thickness direction of the top plate portion and the second tubular portion that is connected to an outer peripheral edge of the presser portion.

14. The cell and biological tissue transporting container according to claim 13, wherein
the outer lid has an opening hole formed inside the presser portion, and
the inner lid is provided with a port that is configured such that the port overlaps the opening hole as viewed in the thickness direction of the top plate portion, an upper end thereof is open to an outside, and a lower end thereof is open to an internal space of the container body.

15. The cell and biological tissue transporting container according to claim 14, wherein
in the inner lid, a first port, a second port, a third port, and a fourth port are provided as the ports,
the first and second ports are provided in the vicinity of the side wall portion of the container body, and the third and fourth ports are provided farther inward in a radial direction of the top plate portion than the first and second ports.

16. The cell and biological tissue transporting container according to claim 15, wherein
at least a central portion of the inner lid is transparent, and
the third and fourth ports are provided at positions displaced from the central portion so as to avoid the central portion.

17. The cell and biological tissue transporting container according to claim 16, wherein
the transparent central portion of the inner lid has a recessed shape that is recessed downward.

18. The cell and biological tissue transporting container according to any one of claims 1 to 17, wherein
the bottom wall portion of the container body is provided with one or more protrusions that protrude upward.

19. The cell and biological tissue transporting container according to claim 18, wherein
the one or more protrusions are in a substantially circular shape or in arc shapes that constitute parts of the same circle as viewed in a vertical direction.

20. The cell and biological tissue transporting container according to any one of claims 1 to 17, wherein
the side wall portion of the container body includes an overhanging portion that overhangs farther inward than a remaining portion as viewed in a vertical direction.

21. The cell and biological tissue transporting container according to any one of claims 1 to 17, wherein
the side wall portion of the container body is provided with a plurality of ribs each of which extends inward and that are disposed radially as viewed in a vertical direction.
